# EUROPÄISCHE PATENTSCHRIFT

(11) **EP 3 237 630 B1**
(45) Veröffentlichungstag und Bekanntmachung des Hinweises auf die Patenterteilung: **31.07.2019**
(21) Anmeldenummer: 15816142.2
(22) Anmeldetag: 16.12.2015
(51) Int. Cl.: C12P 17/04, C07D 307/46

(54) **VERFAHREN ZUR HERSTELLUNG VON (METH)ACRYLSÄUREESTERN VON FURFURYLALKOHOLEN UNTER VERWENDUNG EINER LIPASE**
PROCESS FOR PRODUCING (METH)ACRYLIC ACID ESTERS OF FURFURYL ALCOHOLS EMPLOYING A LIPASE
PROCÉDÉ DE PRODUCTION D'ESTERS D'ACIDE (MÉTH)ACRYLIQUE D'ALCOOLS FURFURYLIQUES UTILISANT UNE LIPASE

(30) Priorität: 22.12.2014 US 201462095097 P
(43) Veröffentlichungstag der Anmeldung: 01.11.2017
(73) Patentinhaber: BASF SE, 67056 Ludwigshafen (DE)
(72) Erfinder: FLECKENSTEIN, Christoph, 63579 Freigericht (DE); BLANCHOT, Mathieu, 67245 Lambsheim (DE); BLANK, Benoit, 68535 Edingen-Neckarhausen (DE); KALLER, Martin, 68163 Mannheim (DE); STENGEL, Ulrik, 69488 Birkenau (DE); MISSKE, Andrea, 67346 Speyer (DE); FLEISCHHAKER, Friederike, 67065 Ludwigshafen (DE); NAIR, Ritesh, 69115 Heidelberg (DE)
(86) Internationale Anmeldenummer: PCT/EP2015/080076
(87) Internationale Veröffentlichungsnummer: WO 2016/102281

(56) Entgegenhaltungen:
- EP-A1- 2 781 961
- US-A1- 2009 018 300

## Beschreibung

Die Erfindung betrifft ein Verfahren zur Herstellung bestimmter Ester auf Basis von Hydroxymethylfurfural (HMF), insbesondere ein Verfahren zur Herstellung von HMF-Acrylat oder HMF-Methacrylat. Weiter betrifft die Erfindung die Ester an sich.

Bei HMF, welches aus Zuckern gewonnen werden kann, handelt es sich um einen vielversprechenden Synthesebaustein aus nachwachsenden Rohstoffen.

In US 2009/0018300 und in Polymer Reprints 2008, 914-915 wird die Herstellung von HMF-Acrylat durch Umsetzung von HMF mit Acryloylchlorid unter Verwendung stöchiometrischer Mengen Triethylamin beschrieben. Diese Synthese weist jedoch einige Nachteile auf, vor allem im Hinblick auf eine Durchführung im großtechnischen Maßstab.

Beispielsweise fällt im Verlauf der Reaktion Triethylammoniumhydrochlorid an, was nicht nur die gegebenenfalls mit Problemen verbundene Handhabung eines Feststoffs erforderlich macht, sondern auch zu Ausbeuteverlusten bei dessen Abtrennung führen kann. Weiterhin von Nachteil sind der Einsatz eines hochreaktiven Säurechlorids und die damit verbundene Freisetzung von Chlorid, da hierdurch die Werkstoffauswahl für Produktionsanlagen eingeschränkt wird und entsprechend resistente Materialien kostspielig sind. Ebenfalls nachteilig ist, dass die Reaktion unter Ausschluss von Feuchtigkeit durchgeführt werden muss, da Säurechloride hydrolyselabil ist. Zudem führt die Umsetzung mit Säurechloriden in der Regel zu relativ dunkel gefärbten Produkten mit einem relativ hohen Chloridgehalt.

US2009/0018300 offenbart weiterhin ein Verfahren zur Herstellung von 5-Butoxymethylfurfurylacrylat durch Umsetzung von Methylacrylat mit 5-Butoxymehtylfurfurylalkohol in Gegenwart einer Lipase.

EP2781961 offenbart die Herstellung von HMF-Methacrylat durch Umsetzung von HMF mit Methacryloylchlorid.

Die Aufgabe der Erfindung liegt in der Bereitstellung eines Verfahrens, mit dem die oben genannten Nachteile überwunden werden und das sich auch im großtechnischen Maßstab durchführen lässt.

Gelöst wird die Aufgabe durch ein Verfahren zur Herstellung einer Verbindung der Formel (I), 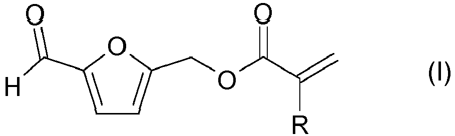 worin R H oder C₁-C₆-Alkyl ist,
durch Umsetzung von mindestens einer Verbindung der Formel (II) 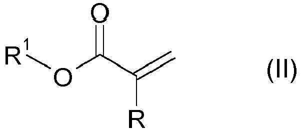
worin R die gleiche Bedeutung wie in der Formel (I) hat und
worin R¹ H, C₁-C₁₂-Alkyl oder C₃-C₁₂-Cycloalkyl ist,
mit einer Verbindung der Formel (III) 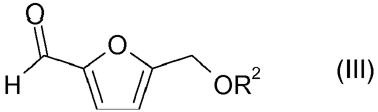
worin R² H oder C(O)R³ ist,
wobei R³ H oder C₁-C₁₂-Alkyl ist,
in Gegenwart von mindestens einem zur Umesterung geeigneten Enzym, wobei eine Lipase (EC 3.1.1.-) als Enzym eingesetzt wird.

Es wurde gefunden, dass die enzymatische Umsetzung hochselektiv verläuft und dass Produkte mit einer hohen Reinheit erhalten werden. Die enzymatische Umsetzung ist auch im großtechnischen Maßstab durchführbar.

Das erfindungsgemäße Verfahren wird in Gegenwart von mindestens einem zur Umesterung geeigneten Enzym, wobei eine Lipase (EC 3.1.1.-) als Enzym eingesetzt wird, durchgeführt, so dass weder die Handhabung noch die Abtrennung eines in stöchiometrischen Mengen anfallenden salzartigen Feststoffs wie Triethylammoniumhydrochlorid erforderlich ist. Des Weiteren geht das erfindungsgemäße Verfahren nicht von hochreaktiven Säurechloriden aus, sondern von Carbonsäuren oder Estern, und stellt damit bezüglich Chloridkorrosion keine besonderen Anforderungen an die Werkstoffe für Produktionsanlagen, sondern kann ohne Schwierigkeiten in gewöhnlichen Apparaturen durchgeführt werden. Ein weiterer Vorteil des erfindungsgemäßen Verfahrens ist, dass es nicht unter striktem Ausschluss von Feuchtigkeit durchgeführt werden muss. Weiterhin von Vorteil ist, auch im Hinblick auf eine gewisse Temperatur- und Säurelabilität von HMF, dass das erfindungsgemäße Verfahren unter milden Reaktionsbedingungen, z.B. bei relativ niedrigen Temperaturen, durchgeführt werden kann. Die enzymatische Umsetzung führt zu deutlich helleren Produkten mit einem deutlich geringeren Chloridgehalt als die Umsetzung mit Säurechloriden. Auch im Vergleich zu klassischen Umesterungen oder Direktveresterungen, die häufig relativ hohen Temperaturen erfordern, liefert das erfindungsgemäße Verfahren deutlich hellere Produkte. Die enzymatische Umsetzung verläuft hochselektiv, und es werden Produkte mit einer hohen Reinheit erhalten.

Erfindungsgemäß ist R in der Formel (I) H oder C₁-C₆-Alkyl.

Beispiele für C₁-C₆-Alkyl sind Methyl, Ethyl, n-Propyl, iso-Propyl, n-Butyl, iso-Butyl, sec-Butyl, tert-Butyl, n-Pentyl, iso-Pentyl, sec-Pentyl, tert-Pentyl, neo-Pentyl, Hexyl.

In einer bevorzugten Ausführungsform der Erfindung ist R in der Formel (I) H oder C₁-C₄-Alkyl.

Beispiele für C₁-C₄-Alkyl sind Methyl, Ethyl, n-Propyl, iso-Propyl, n-Butyl, iso-Butyl, sec-Butyl, tert-Butyl.

In einer besonders bevorzugten Ausführungsform der Erfindung ist R in der Formel (I) H oder CH₃.

In einer ganz besonders bevorzugten Ausführungsform der Erfindung ist R in der Formel (I) H.

In einer weiteren ganz besonders bevorzugten Ausführungsform der Erfindung ist R in der Formel (I) CH₃.

In einer weiteren Ausführungsform der Erfindung ist R in der Formel (I) C₁-C₆-Alkyl.

In einer weiteren Ausführungsform der Erfindung ist R in der Formel (I) C₁-C₄-Alkyl.

Erfindungsgemäß hat R in der Formel (II) die gleiche Bedeutung wie R in der Formel (I).

Bevorzugt ist für R in der Formel (II) die Bedeutung, die für R in der Formel (I) bevorzugt ist.

Besonders bevorzugt ist für R in der Formel (II) die Bedeutung, die für R in der Formel (I) besonders bevorzugt ist.

Ganz besonders bevorzugt ist für R in der Formel (II) die Bedeutung, die für R in der Formel (I) ganz besonders bevorzugt ist.

Erfindungsgemäß ist R¹ in der Formel (II) H, C₁-C₁₂-Alkyl oder C₃-C₁₂-Cycloalkyl.

Beispiele für C₁-C₁₂-Alkyl sind Methyl, Ethyl, n-Propyl, iso-Propyl, n-Butyl, iso-Butyl, sec-Butyl, tert-Butyl, n-Pentyl, iso-Pentyl, sec-Pentyl, tert-Pentyl, neo-Pentyl, Hexyl, Heptyl, Octyl, insbesondere 2-Ethylhexyl, Nonyl, insbesondere Isononyl, Decyl, insbesondere 2-Propylheptyl, Undecyl, Dodecyl.

Beispiele für C₃-C₁₂-Cycloalkyl sind Cyclopropyl, Cyclobutyl, Cyclopentyl, Cyclohexyl, Cycloheptyl, Cyclooctyl, Cyclononyl, Cyclodecyl, Cycloundecyl, Cyclododecyl.

In einer bevorzugten Ausführungsform der Erfindung ist R¹ in der Formel (II) C₁-C₁₂-Alkyl.

In einer besonderen Ausführungsform der Erfindung ist R¹ in der Formel (II) Methyl, Ethyl, n-Propyl, iso-Propyl, n-Butyl, iso-Butyl, sec-Butyl, tert-Butyl oder 2-Ethylhexyl.

In einer weiteren bevorzugten Ausführungsform der Erfindung ist R¹ in der Formel (II) H oder C₁-C₄-Alkyl.

Beispiele für C₁-C₄-Alkyl sind Methyl, Ethyl, n-Propyl, iso-Propyl, n-Butyl, iso-Butyl, sec-Butyl, tert-Butyl.

In einer besonders bevorzugten Ausführungsform der Erfindung ist R¹ in der Formel (II) C₁-C₄-Alkyl.

In einer ganz besonders bevorzugten Ausführungsform der Erfindung ist R¹ in der Formel (II) CH₃ oder CH₂CH₃, insbesondere CH₃.

In einer weiteren Ausführungsform der Erfindung ist R¹ in der Formel (II) H.

In einer weiteren bevorzugten Ausführungsform der Erfindung ist R¹ in der Formel (II) C₁-C₁₂-Alkyl oder C₅-C₇-CyCloalkyl.

In einer weiteren besonderen Ausführungsform der Erfindung ist R¹ in der Formel (II) Methyl, Ethyl, n-Propyl, iso-Propyl, n-Butyl, iso-Butyl, sec-Butyl, tert-Butyl, 2-Ethylhexyl oder Cyclohexyl.

In einer weiteren bevorzugten Ausführungsform der Erfindung ist R¹ in der Formel (II) H, C₁-C₄-Alkyl oder Cyclohexyl.

In einer weiteren besonders bevorzugten Ausführungsform der Erfindung ist R¹ in der Formel (II) C₁-C₄-Alkyl oder Cyclohexyl.

In einer weiteren Ausführungsform der Erfindung ist R¹ in der Formel (II) C₈-C₁₀-Alkyl, bevorzugt 2-Ethylhexyl, Isononyl oder 2-Propylheptyl, besonders bevorzugt 2-Ethylhexyl.

In einer weiteren Ausführungsform der Erfindung ist R¹ in der Formel (II) C₅-C₇-Cycloalkyl, bevorzugt Cyclopentyl oder Cyclohexyl, besonders bevorzugt Cyclohexyl.

Beispiele für Verbindungen der Formel (II) sind Methylacrylat, Ethylacrylat, n-Propylacrylat, iso-Propylacrylat, n-Butylacrylat, iso-Butylacrylat, sec-Butylacrylat, tert-Butylacrylat, n-Pentylacrylat, iso-Pentylacrylat, sec-Pentylacrylat, tert-Pentylacrylat, neo-Pentylacrylat, Hexylacrylat, Heptylacrylat, Octylacrylat, insbesondere 2-Ethylhexylacrylat, Nonylacrylat, insbesondere Isononylacrylat, Decylacrylat, insbesondere 2-Propylheptylacrylat, Undecylacrylat, Dodecylacrylat.

Weitere Beispiele für Verbindungen der Formel (II) sind Methylmethacrylat, Ethylmethacrylat, n-Propylmethacrylat, iso-Propylmethacrylat, n-Butylmethacrylat, iso-Butylmethacrylat, sec-Butylmethacrylat, tert-Butylmethacrylat, n-Pentylmethacrylat, iso-Pentylmethacrylat, sec-Pentylmethacrylat, tert-Pentylmethacrylat, neo-Pentylmethacrylat, Hexylmethacrylat, Heptylmethacrylat, Octylmethacrylat, insbesondere 2-Ethylhexylmethacrylat, Nonylmethacrylat, insbesondere Isononylmethacrylat, Decylmethacrylat, insbesondere 2-Propylheptylmethacrylat, Undecylmethacrylat, Dodecylmethacrylat.

Weitere Beispiele für Verbindungen der Formel (II) sind Acrylsäure, Methacrylsäure.

In einer bevorzugten Ausführungsform der Erfindung wird Methylacrylat, Ethylacrylat, n-Propylacrylat, iso-Propylacrylat, n-Butylacrylat, iso-Butylacrylat, sec-Butylacrylat, tert-Butylacrylat, 2-Ethylhexylacrylat, Methylmethacrylat, Ethylmethacrylat, n-Propylmethacrylat, iso-Propylmethacrylat, n-Butylmethacrylat, iso-Butylmethacrylat, sec-Butylmethacrylat, tert-Butylmethacrylat oder 2-Ethylhexylmethacrylat als Verbindung der Formel (II) eingesetzt.

In einer besonders bevorzugten Ausführungsform der Erfindung wird Methylacrylat, Ethylacrylat, Methylmethacrylat oder Ethylmethacrylat als Verbindung der Formel (II) eingesetzt.

In einer ganz besonders bevorzugten Ausführungsform der Erfindung wird Methylacrylat als Verbindung der Formel (II) eingesetzt.

In einer weiteren ganz besonders bevorzugten Ausführungsform der Erfindung wird Methylmethacrylat als Verbindung der Formel (II) eingesetzt.

In einer weiteren Ausführungsform der Erfindung wird Acrylsäure als Verbindung der Formel (II) eingesetzt.

In einer weiteren Ausführungsform der Erfindung wird Methacrylsäure als Verbindung der Formel (II) eingesetzt.

Erfindungsgemäß wird mindestens eine Verbindung der Formel (II) eingesetzt. Bevorzugt werden eine bis drei Verbindungen der Formel (II) eingesetzt. Besonders bevorzugt werden eine oder zwei Verbindungen der Formel (II) eingesetzt. Ganz besonders bevorzugt wird eine (1) Verbindung der Formel (II) eingesetzt.

Die Verbindungen der Formel (II) sind kommerziell erhältlich oder können nach dem Fachmann bekannten Methoden hergestellt werden.

Verbindungen der Formel (II), in denen R¹ nicht H ist, können z.B. aus Verbindungen der Formel (II), in denen R¹ H ist, durch Veresterung hergestellt werden, beispielsweise unter Verwendung eines Alkohols in Gegenwart einer Säure als Katalysator.

Beispielsweise können Verbindungen der Formel (II), in denen R¹ Methyl, Ethyl oder n-Butyl ist, aus Verbindungen der Formel (II), in denen R¹ H ist, durch Veresterung unter Verwendung von Methanol, Ethanol oder n-Butanol als Alkohol in Gegenwart einer Säure als Katalysator hergestellt werden.

Verbindungen der Formel (II), in denen R¹ tert-Butyl ist, können z.B. aus Verbindungen der Formel (II), in denen R¹ H ist, durch Umsetzung mit Isobuten in Gegenwart einer Säure als Katalysator hergestellt werden.

Verbindungen der Formel (II), in denen R¹ nicht H ist, können z.B. aus Verbindungen der Formel (II), in denen R¹ ebenfalls nicht H ist, durch Umesterung hergestellt werden, beispielsweise unter Verwendung eines Alkohols in Gegenwart einer Säure oder einer Base als Katalysator.

Verbindungen der Formel (II), in denen R¹ H ist, können z.B. aus Verbindungen der Formel (II), in denen R¹ nicht H ist, durch Hydrolyse hergestellt werden, beispielsweise in Gegenwart einer Säure oder einer Base als Katalysator.

Erfindungsgemäß ist R² in der Formel (III) H oder C(O)R³.

In einer bevorzugten Ausführungsform der Erfindung ist R² in der Formel (III) H. Verbindungen der Formel (III), in denen R² H ist, werden als Verbindungen der Formel (IIIa) bezeichnet:

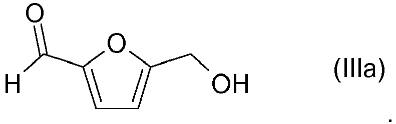

In einer weiteren Ausführungsform der Erfindung ist R² in der Formel (III) C(O)R³. Verbindungen der Formel (III), in denen R² C(O)R³ ist, werden als Verbindungen der Formel (IIIb) bezeichnet: 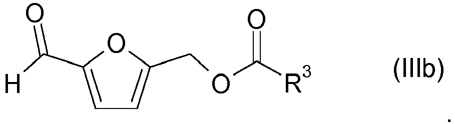 Erfindungsgemäß ist R³ H oder C₁-C₁₂-Alkyl.

Beispiele für C₁-C₁₂-Alkyl sind Methyl, Ethyl, n-Propyl, iso-Propyl, n-Butyl, iso-Butyl, sec-Butyl, tert-Butyl, n-Pentyl, iso-Pentyl, sec-Pentyl, tert-Pentyl, neo-Pentyl, Hexyl, Heptyl, Octyl, insbesondere 2-Ethylhexyl, Nonyl, insbesondere Isononyl, Decyl, insbesondere 2-Propylheptyl, Undecyl, Dodecyl.

In einer bevorzugten Ausführungsform der Erfindung ist R³ H oder C₁-C₈-Alkyl.

Beispiele für C₁-C₈-Alkyl sind Methyl, Ethyl, n-Propyl, iso-Propyl, n-Butyl, iso-Butyl, sec-Butyl, tert-Butyl oder 2-Ethylhexyl.

In einer besonders bevorzugten Ausführungsform der Erfindung ist R³ H oder C₁-C₄-Alkyl.

Beispiele für C₁-C₄-Alkyl sind Methyl, Ethyl, n-Propyl, iso-Propyl, n-Butyl, iso-Butyl, sec-Butyl, tert-Butyl.

In einer ganz besonders bevorzugten Ausführungsform der Erfindung ist R³ H oder CH₃, insbesondere CH₃.

In einer weiteren besonderen Ausführungsform der Erfindung ist R³ H.

Die Verbindungen der Formel (III) sind kommerziell erhältlich (z.B. Aldrich) oder können nach in der Literatur bekannten Methoden hergestellt werden (B. Kim et al., Ind. Eng. Chem. Res. 2014, 53, 4633-4641; R.-J. van Putten et al., Chem. Rev. 2013, 113, 1499-1597; EP 1958944; DE 3309564).

Geeignete Methoden zur Herstellung der Verbindungen der Formel (IIIb), insbesondere geeignete Formylierungs- oder Acylierungsmethoden zur Einführung der Gruppe C(O)R³, sind dem Fachmann bekannt.

Die mindestens eine Verbindung der Formel (II) und die Verbindung der Formel (III) werden im Allgemeinen in einem Molverhältnis von 1:10 bis 25:1, bevorzugt von 1:1 bis 20:1, besonders bevorzugt von 5:1 bis 15:1, ganz besonders bevorzugt von 8:1 bis 12:1 eingesetzt.

In einer Ausführungsform der Erfindung ist R¹ in der Formel (II) H, wenn R² in der Formel (III) C(O)R³ ist. In einer weiteren Ausführungsform der Erfindung ist R² in der Formel (III) C(O)R³, wenn R¹ in der Formel (II) H ist. In einer weiteren Ausführungsform der Erfindung ist R¹ in der Formel (II) H und R² in der Formel (III) C(O)R³.

In einer Ausführungsform der Erfindung ist R¹ in der Formel (II) nicht H, wenn R² in der Formel (III) H ist. In einer weiteren Ausführungsform der Erfindung ist R² in der Formel (III) H, wenn R¹ in der Formel (II) nicht H ist. In einer weiteren Ausführungsform der Erfindung ist R¹ in der Formel (II) nicht H und R² in der Formel (III) H.

Erfindungsgemäß wird mindestens ein zur Umesterung geeignetes Enzym eingesetzt, wobei eine Lipase (EC 3.1.1.-) als Enzym eingesetzt wird. Beispiele für geeignete Lipasen (EC 3.1.1.-) sind Triacylglycerollipasen (EC 3.1.1.3).

Bevorzugt wird eine Triacylglycerollipase (EC 3.1.1.3) als Enzym eingesetzt.

Besonders bevorzugt sind Novozym® 435 (Lipase aus Candida antarctica B) oder Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas, ganz besonders bevorzugt sind Lipase aus Candida antarctica B oder Lipase aus Burkholderia sp., insbesondere bevorzugt ist Lipase aus Candida antarctica B.

Die Enzyme sind kommerziell erhältlich (z.B. Novozym® 435) oder können nach dem Fachmann bekannten Methoden erhalten werden.

Das mindestens eine Enzym kann in freier Form oder in immobilisierter Form eingesetzt werden. In einer Ausführungsform der Erfindung wird das mindestens eine Enzym in freier Form eingesetzt. In einer weiteren Ausführungsform der Erfindung wird das mindestens eine Enzym in immobilisierter Form eingesetzt.

Das mindestens eine Enzym kann chemisch oder physikalisch immobilisiert sein. Geeignete Träger und geeignete Methoden zur Immobilisierung sind dem Fachmann bekannt.

Ein Beispiel für einen geeigneten Träger ist ein Acrylharz. Ein weiteres Beispiel für einen geeigneten Träger ist Lewatit®. Ein weiteres Beispiel für einen geeigneten Träger ist Lewatit® VP OC 1600. Lewatit® VP OC 1600 ist ein makroporöses, Divinylbenzol-vernetztes Polymer in sphärischer Perlenform auf Basis von Methacrylat. Ein weiteres Beispiel für einen geeigneten Träger ist demnach ein makroporöses, Divinylbenzol-vernetztes Polymer in sphärischer Perlenform auf Basis von Methacrylat.

In einer bevorzugten Ausführungsform der Erfindung wird eine Lipase aus Candida antarctica B als Enzym eingesetzt.

In einer besonders bevorzugten Ausführungsform der Erfindung wird eine Lipase aus Candida antarctica B in immobilisierter Form als Enzym eingesetzt.

In einer besonderen Ausführungsform der Erfindung wird eine Lipase aus Candida antarctica B in immobilisierter Form als Enzym eingesetzt, wobei als Träger ein Acrylharz verwendet wird. In einer weiteren besonderen Ausführungsform der Erfindung wird eine Lipase aus Candida antarctica B in immobilisierter Form als Enzym eingesetzt, wobei als Träger Lewatit®, insbesondere Lewatit® VP OC 1600, verwendet wird. In einer weiteren besonderen Ausführungsform der Erfindung wird eine Lipase aus Candida antarctica B in immobilisierter Form als Enzym eingesetzt, wobei als Träger ein makroporöses, Divinylbenzol-vernetztes Polymer in sphärischer Perlenform auf Basis von Methacrylat verwendet wird.

In einer ganz besonders bevorzugten Ausführungsform der Erfindung wird Novozym® 435 als Enzym eingesetzt.

Das mindestens eine Enzym wird im Allgemeinen in einer Menge im Bereich von 0,1 bis 15 Gew.-%, bevorzugt 1 bis 10 Gew.-%, besonders bevorzugt 5 bis 9 Gew.-%, ganz besonders bevorzugt 6 bis 8 Gew.-%, bezogen auf die Menge an eingesetzter Verbindung der Formel (III), eingesetzt.

In einer bevorzugten Ausführungsform der Erfindung wird das mindestens eine Enzym in immobilisierter Form und in einer Menge im Bereich von 0,1 bis 15 Gew.-%, bevorzugt 1 bis 10 Gew.-%, besonders bevorzugt 5 bis 9 Gew.-%, ganz besonders bevorzugt 6 bis 8 Gew.-%, bezogen auf die Menge an eingesetzter Verbindung der Formel (III), eingesetzt.

In einer besonders bevorzugten Ausführungsform der Erfindung wird Novozym® 435 als Enzym und in einer Menge im Bereich von 0,1 bis 15 Gew.-%, bevorzugt 1 bis 10 Gew.-%, besonders bevorzugt 5 bis 9 Gew.-%, ganz besonders bevorzugt 6 bis 8 Gew.-%, bezogen auf die Menge an eingesetzter Verbindung der Formel (III), eingesetzt.

SEQ ID NO: 1 bezieht sich auf die folgende Aminosäuresequenz:

MKLLSLTGVAGVLATCVAATPLVKRLPSGSDPAFSQPKSVLDAGLTCQGASPSSVSKPILL-
VPGTGTTGPQSFDSNWIPLSTQLGYTPCWISPPPFMLNDTQVNTEYMVNAITALYAGSGNN-
KLPVLTWSQGGLVAQWGLTFFPSIRSKVDRLMAFAPDYKGTVLAGPLDALAV-
SAPSVWQQTTGSALTTALRNAGGLTQIVPTTNLYSATDEIVQPQVSNSPLDSSYLFNG-
KNVQAQAVCGPLFVIDHAGSLTSQFSYVVGRSALRSTTGQARSADYGITDCNPLPANDLT-
PEQKVAAAALLAPAAAAIVAGPKQNCEPDLMPYARPFAVGKRTCSGIVTP

Die N-terminalen 25 Aminosäuren der SEQ ID NO: 1 können als Prä-Propeptid angesehen werden, welches eine Sequenz eines Signalpeptids und eine Sequenz eines Propeptids enthalten kann. Daher kann die Sequenz gegebenenfalls auch bei der Aminosäure in Position 26 beginnen.

Bevorzugt umfasst das erfindungsgemäß eingesetzte Enzym eine Aminosäuresequenz mit mindestens 80% Homologie zu SEQ ID NO: 1, mehr bevorzugt mit mindestens 85% Homologie zu SEQ ID NO: 1, noch mehr bevorzugt mit mindestens 90% Homologie zu SEQ ID NO: 1, noch mehr bevorzugt mit mindestens 95% Homologie zu SEQ ID NO: 1, noch mehr bevorzugt mit mindestens 98% Homologie zu SEQ ID NO: 1, noch mehr bevorzugt mit mindestens 99% Homologie zu SEQ ID NO: 1, und insbesondere eine Aminosäuresequenz der SEQ ID NO: 1. Alternativ ist das erfindungsgemäß eingesetzte Enzym bevorzugt ein funktionelles Derivat davon.

Besonders bevorzugt besteht das erfindungsgemäß eingesetzte Enzym aus einer Aminosäuresequenz mit mindestens 80% Homologie zu SEQ ID NO: 1, mehr bevorzugt mit mindestens 85% Homologie zu SEQ ID NO: 1, noch mehr bevorzugt mit mindestens 90% Homologie zu SEQ ID NO: 1, noch mehr bevorzugt mit mindestens 95% Homologie zu SEQ ID NO: 1, noch mehr bevorzugt mit mindestens 98% Homologie zu SEQ ID NO: 1, noch mehr bevorzugt mit mindestens 99% Homologie zu SEQ ID NO: 1, und insbesondere aus einer Aminosäuresequenz der SEQ ID NO: 1. Alternativ ist das erfindungsgemäß eingesetzte Enzym besonders bevorzugt ein funktionelles Derivat davon.

Bevorzugt zeigt das erfindungsgemäß eingesetzte Enzym mindestens 10%, mehr bevorzugt mindestens 20%, noch mehr bevorzugt mindestens 30%, noch mehr bevorzugt mindestens 40%, noch mehr bevorzugt mindestens 50%, noch mehr bevorzugt mindestens 60%, noch mehr bevorzugt mindestens 70%, noch mehr bevorzugt mindestens 80%, noch mehr bevorzugt mindestens 90%, oder sogar 100% oder mehr der Umesterungsaktivität eines Enzyms umfassend, vorzugsweise bestehend aus, eine(r) Aminosäuresequenz der SEQ ID NO: 1.

SEQ ID NO: 2 bezieht sich auf die folgende Aminosäuresequenz:

-
-
-
-
-


Auch bevorzugt umfasst das erfindungsgemäß eingesetzte Enzym eine Aminosäuresequenz mit mindestens 80% Homologie zu SEQ ID NO: 2, mehr bevorzugt mit mindestens 85% Homologie zu SEQ ID NO: 2, noch mehr bevorzugt mit mindestens 90% Homologie zu SEQ ID NO: 2, noch mehr bevorzugt mit mindestens 95% Homologie zu SEQ ID NO: 2, noch mehr bevorzugt mit mindestens 98% Homologie zu SEQ ID NO: 2, noch mehr bevorzugt mit mindestens 99% Homologie zu SEQ ID NO: 2, und insbesondere eine Aminosäuresequenz der SEQ ID NO: 2. Alternativ ist das erfindungsgemäß eingesetzte Enzym bevorzugt ein funktionelles Derivat davon.

Auch besonders bevorzugt besteht das erfindungsgemäß eingesetzte Enzym aus einer Aminosäuresequenz mit mindestens 80% Homologie zu SEQ ID NO: 2, mehr bevorzugt mit mindestens 85% Homologie zu SEQ ID NO: 2, noch mehr bevorzugt mit mindestens 90% Homologie zu SEQ ID NO: 2, noch mehr bevorzugt mit mindestens 95% Homologie zu SEQ ID NO: 2, noch mehr bevorzugt mit mindestens 98% Homologie zu SEQ ID NO: 2, noch mehr bevorzugt mit mindestens 99% Homologie zu SEQ ID NO: 2, und insbesondere aus einer Aminosäuresequenz der SEQ ID NO: 2. Alternativ ist das erfindungsgemäß eingesetzte Enzym besonders bevorzugt ein funktionelles Derivat davon.

Auch bevorzugt zeigt das erfindungsgemäß eingesetzte Enzym mindestens 10%, mehr bevorzugt mindestens 20%, noch mehr bevorzugt mindestens 30%, noch mehr bevorzugt mindestens 40%, noch mehr bevorzugt mindestens 50%, noch mehr bevorzugt mindestens 60%, noch mehr bevorzugt mindestens 70%, noch mehr bevorzugt mindestens 80%, noch mehr bevorzugt mindestens 90%, oder sogar 100% oder mehr der Umesterungsaktivität eines Enzyms umfassend, vorzugsweise bestehend aus, eine(r) Aminosäuresequenz der SEQ ID NO: 2.

In einer besonderen Ausführungsform besteht das erfindungsgemäß eingesetzte Enzym aus der Aminosäuresequenz, die beschrieben ist in Structure 1994, Vol 2, No 4, Seiten 293-308 (Jonas Uppenberg, Mogens Trier Hansen, Shamkant Patkar, T Alwyn Jones: The sequence, crystal structure determination and refinement of two crystal forms of lipase B from Candida antarctica). Die Aminosäuresequenz ist auf Seite 294, Fig. 1 und ihrem Begleittext, dieses Artikels offenbart. Wie in dem Begleittext zu Fig. 1 beschrieben ist, werden die N-terminalen 25 Aminosäuren, d.h. die Aminosäuren -25 bis -1, als Prä-Propeptid bezeichnet. In einer Ausführungsform ist das Enzym das auf Seite 294, Fig. 1 und ihrem Begleittext, offenbarte Volllängenpolypeptid, welches auch die Aminosäuren -25 bis - 1 enthält. In einer anderen Ausführungsform besteht das Enzym aus der auf Seite 294, Fig. 1 und ihrem Begleittext, offenbarten Aminosäuresequenz, welche die Aminosäuren -25 bis -1 nicht enthält. Dem Fachmann ist bekannt, dass das Enzym auch ohne das in Fig. 1 auf Seite 294 abgebildete C-terminale OPA eingesetzt werden kann.

Der Begriff "Homologie" bedeutet Sequenzhomologie und/oder dreidimensionale (3D) Strukturhomologie. Vorzugsweise bedeutet der Begriff "Homologie" Sequenzhomologie.

Der Begriff "funktionelles Derivat davon" bezieht sich auf ein Enzym, das mindestens 10%, mehr bevorzugt mindestens 20%, noch mehr bevorzugt mindestens 30%, noch mehr bevorzugt mindestens 40%, noch mehr bevorzugt mindestens 50%, noch mehr bevorzugt mindestens 60%, noch mehr bevorzugt mindestens 70%, noch mehr bevorzugt mindestens 80%, noch mehr bevorzugt mindestens 90%, oder sogar 100% oder mehr der Umesterungsaktivität eines Enzyms umfassend, vorzugsweise bestehend aus, eine(r) Aminosäuresequenz der SEQ ID NO: 1 bzw. SEQ ID NO: 2 zeigt.

Dem Fachmann ist bekannt, dass das erfindungsgemäß eingesetzte Enzym gegebenenfalls eine oder mehrere posttranslationale Modifikation(en) enthalten kann.

Dem Fachmann ist bekannt, dass das erfindungsgemäß eingesetzte Enzym gegebenenfalls mit einem oder mehreren anderen Molekül(en) konjugiert oder an ein oder mehrere andere(s) Molekül(e) gebunden sein kann. Beispiele für solche anderen Moleküle umfassen fluoreszente Moleküle. Ein Beispiel für ein Fluoreszenzderivatisierungsreagenz ist ortho-Phthaldialdehyd (OPA).

Dem Fachmann ist bekannt, dass das erfindungsgemäß eingesetzte Enzym gegebenenfalls mit verschiedenen Isotopen, wie ²H, ³H, ¹³C, ¹⁴C, ³²P und/oder ³⁵S, markiert sein kann.

Gegebenenfalls wird das erfindungsgemäße Verfahren in Gegenwart von einem oder mehreren weiteren Additiven durchgeführt.

Beispiele für weitere Additive sind Stabilisatoren, Molekularsiebe oder Zeolithe. Geeignete Stabilisatoren, Molekularsiebe oder Zeolithe sind dem Fachmann bekannt. Dem Fachmann ist auch bekannt, in welchen Mengen Stabilisatoren, Molekularsiebe oder Zeolithe eingesetzt werden können.

Gegebenenfalls wird das erfindungsgemäße Verfahren in Gegenwart von einem oder mehreren Stabilisatoren durchgeführt.

Wird das erfindungsgemäße Verfahren in Gegenwart von einem oder mehreren Stabilisatoren durchgeführt, so werden bevorzugt ein bis drei Stabilisatoren, besonders bevorzugt ein oder zwei Stabilisatoren, ganz besonders bevorzugt ein (1) Stabilisator eingesetzt.

Beispiele für geeignete Stabilisatoren sind N-Oxide (Nitroxyl- oder N-Oxyl-Radikale), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethylpiperidin-N-oxyl, 2,2,6,6-Tetramethylpiperidin-N-oxyl, Bis(1-oxyl-2,2,6,6-tetramethylpiperidin-4-yl)sebacat, 4,4',4"-Tris(2,2,6,6-tetramethylpiperidin-N-oxyl)-phosphit oder 3-Oxo-2,2,5,5-tetramethylpyrrolidin-N-oxyl; ein- oder mehrwertige Phenole, die ggf. eine oder mehrere Alkylgruppen aufweisen, wie z. B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z. B. Hydrochinon, Hydrochinonmonomethylether, 2-Methylhydrochinon oder 2,5-Di-tert.-butylhydrochinon; Hydroxyphenole, wie beispielsweise Brenzcatechin (1,2-Dihydroxybenzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-butyl-4-methoxyphenol; Tocopherole, wie z. B. α-Tocopherol sowie 2,3-Dihydro-2,2-dimethyl-7-hydroxybenzofuran (2,2-Dimethyl-7-hydroxycumaran), aromatische Amine, wie z. B. N,N-Diphenylamin oder N-Nitrosodiphenylamin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z. B. N,N-Diethylhydroxylamin, Imine, wie z. B. Methylethylimin oder Methylenviolett, Sulfonamide, wie z. B. N-Methyl-4-toluolsulfonamid oder N-tert.-butyl-4-toluolsulfonamid, Oxime, wie Aldoxime, Ketoxime oder Amidoxime, wie z. B. Diethylketoxim, Methylethylketoxim oder Salicylaldoxim, phosphorhaltige Verbindungen, wie z. B. Triphenylphosphin, Triphenylphosphit, Triethylphosphit, Hypophsophorige Säure oder Alkylester der Phosphorigen Säuren; schwefelhaltige Verbindungen wie z. B. Diphenylsulfid oder Phenothiazin; Metallsalze, wie Kupfer- oder Mangan-, Cer-, Nickel-, Chromsalze, beispielsweise -chloride, -sulfate, -salicylate, -tosylate, -acrylate oder -acetate, wie z. B. Kupferacetat, Kupfer(II)chlorid, Kupfersalicylat, Cer(III)acetat oder Cer(III)ethylhexanoat.

Bevorzugte Stabilisatoren sind ausgewählt aus der Gruppe bestehend aus Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, Bis(1-oxyl-2,2,6,6-tetramethylpiperidin-4-yl)sebacat, 2-tert.-Butylphenol, 4-tert.-Butylphenol, 2,4-Di-tert.-butylphenol, 2-tert.-Butyl-4-methylphenol, 6-tert.-Butyl-2,4-dimethylphenol, 2,6-Di-tert.-butyl-4-methylphenol, 2-Methyl-4-tert.-butylphenol, Hypophosphorige Säure, Kupferacetat, Kupfer(II)chlorid, Kupfersalicylat und Cer(III)acetat.

Besonders bevorzugte Stabilisatoren sind ausgewählt aus der Gruppe bestehend aus Hydrochinonmonomethylether, Phenothiazin, 2-tert.-Butyl-4-methylphenol, 6-tert.-Butyl-2,4-dimethylphenol, 4-Hydroxy-2,2,6,6-tetramethylpiperidin-N-oxyl und 4-Oxo-2,2,6,6-tetramethylpiperidin-N-oxyl.

Ganz besonders bevorzugte Stabilisatoren sind ausgewählt aus der Gruppe bestehend aus Hydrochinonmonomethylether und Phenothiazin.

Ein insbesondere bevorzugter Stabilisator ist Hydrochinonmonomethylether.

Wird das erfindungsgemäße Verfahren in Gegenwart von einem oder mehreren, bevorzugt einem bis drei, besonders bevorzugt einem oder zwei Stabilisatoren, ganz besonders bevorzugt einem (1) Stabilisator durchgeführt, so wird jeder Stabilisator im Allgemeinen in einer Menge im Bereich von 1 bis 10000 ppm, bevorzugt 10 bis 5000 ppm, besonders bevorzugt 30 bis 2500 ppm, ganz besonders bevorzugt 50 bis 1500 ppm, bezogen auf die Menge an eingesetzter Verbindung der Formel (II), eingesetzt.

In einer besonderen Ausführungsform der Erfindung wird das erfindungsgemäße Verfahren in Gegenwart von Hydrochinonmonomethylether als Stabilisator durchgeführt, und der Stabilisator Hydrochinonmonomethylether wird im Allgemeinen in einer Menge im Bereich von 1 bis 10000 ppm, bevorzugt 10 bis 5000 ppm, besonders bevorzugt 30 bis 2500 ppm, ganz besonders bevorzugt 50 bis 1500 ppm, bezogen auf die Menge an eingesetzter Verbindung der Formel (II), eingesetzt.

Ein Vorteil des Einsatzes von einem oder mehreren Stabilisatoren, insbesondere des Einsatzes von Hydrochinonmonomethylether als Stabilisator, ist, dass die Polymerisation von eingesetzter Verbindung der Formel (II) und hergestellter Verbindung der Formel (I) verhindert wird.

Gegebenenfalls wird das erfindungsgemäße Verfahren in Gegenwart von einem oder mehreren Molekularsieben durchgeführt.

Wird das erfindungsgemäße Verfahren in Gegenwart von einem oder mehreren Molekularsieben durchgeführt, so werden bevorzugt ein bis drei Molekularsiebe, besonders bevorzugt ein oder zwei Molekularsiebe, ganz besonders bevorzugt ein (1) Molekularsieb eingesetzt.

Beispiele für geeignete Molekularsiebe sind Molekularsiebe mit einer Porengröße im Bereich von 3 bis 10 Angstroem, bevorzugt 3 bis 7 Angstroem, besonders bevorzugt 4 bis 6 Angstroem, ganz besonders bevorzugt 5 Angstroem.

Wird das erfindungsgemäße Verfahren in Gegenwart von einem oder mehreren, bevorzugt einem bis drei, besonders bevorzugt einem oder zwei Molekularsieben, ganz besonders bevorzugt einem (1) Molekularsieb durchgeführt, so werden jedes Molekularsieb und die Verbindung der Formel (III) im Allgemeinen in einem Gewichtsverhältnis von 1:10 bis 10:1, bevorzugt von 1:1 bis 5:1, besonders bevorzugt von 1,5:1 bis 4:1, ganz besonders bevorzugt von 2:1 bis 3:1 eingesetzt.

In einer besonderen Ausführungsform der Erfindung wird das erfindungsgemäße Verfahren in Gegenwart von einem Molekularsieb mit einer Porengröße von 5 Angstroem durchgeführt, und das Molekularsieb mit einer Porengröße von 5 Angstroem und die Verbindung der Formel (III) werden im Allgemeinen in einem Gewichtsverhältnis von 1:10 bis 10:1, bevorzugt von 1:1 bis 5:1, besonders bevorzugt von 1,5:1 bis 4:1, ganz besonders bevorzugt von 2:1 bis 3:1 eingesetzt.

Ein Vorteil des Einsatzes von einem oder mehreren Molekularsieben, insbesondere des Einsatzes von einem Molekularsieb mit einer Porengröße von 5 Angstroem, ist, dass ein höherer Umsatz von eingesetzter Verbindung der Formel (III) zu hergestellter Verbindung der Formel (I) erzielt wird. Gegebenenfalls eingesetztes Molekularsieb, insbesondere gegebenenfalls eingesetztes Molekularsieb mit einer Porengröße von 5 Angstroem, kann freigesetzten Alkohol, z.B. freigesetztes Methanol, aufnehmen und so aus dem Gleichgewicht entfernen.

In einer ganz besonderen Ausführungsform der Erfindung wird das erfindungsgemäße Verfahren in Gegenwart von Hydrochinonmonomethylether als Stabilisator und in Gegenwart von einem Molekularsieb mit einer Porengröße von 5 Angstroem durchgeführt.

In einer weiteren ganz besonderen Ausführungsform der Erfindung wird das erfindungsgemäße Verfahren in Gegenwart von Hydrochinonmonomethylether als Stabilisator und in Gegenwart von einem Molekularsieb mit einer Porengröße von 5 Angstroem durchgeführt, wobei der Stabilisator Hydrochinonmonomethylether im Allgemeinen in einer Menge im Bereich von 1 bis 10000 ppm, bevorzugt 10 bis 5000 ppm, besonders bevorzugt 30 bis 2500 ppm, ganz besonders bevorzugt 50 bis 1500 ppm, bezogen auf die Menge an eingesetzter Verbindung der Formel (II), eingesetzt wird, und wobei das Molekularsieb mit einer Porengröße von 5 Angstroem und die Verbindung der Formel (III) im Allgemeinen in einem Gewichtsverhältnis von 1:10 bis 10:1, bevorzugt von 1:1 bis 5:1, besonders bevorzugt von 1,5:1 bis 4:1, ganz besonders bevorzugt von 2:1 bis 3:1 eingesetzt werden.

Im Allgemeinen wird das erfindungsgemäße Verfahren bei Temperaturen im Bereich von 0 bis 100 °C, bevorzugt 10 bis 80 °C, besonders bevorzugt 20 bis 60 °C, ganz besonders bevorzugt 30 bis 50 °C durchgeführt.

Vorzugsweise werden mindestens eine Verbindung der Formel (II) und eine Verbindung der Formel (III) in Gegenwart von mindestens einem Enzym und gegebenenfalls in Gegenwart von einem oder mehreren weiteren Additiven über einen Zeitraum von 1 bis 96 h, besonders bevorzugt 12 bis 72 h, ganz besonders bevorzugt 24 bis 60 h miteinander umgesetzt.

In einer besonders bevorzugten Ausführungsform der Erfindung fungiert die mindestens eine eingesetzte Verbindung der Formel (II) als Lösungsmittel.

In einer ganz besonders bevorzugten Ausführungsform der Erfindung fungiert Methylacrylat als Lösungsmittel.

In einer weiteren ganz besonders bevorzugten Ausführungsform der Erfindung fungiert Methylmethacrylat als Lösungsmittel.

In einer weiteren Ausführungsform der Erfindung wird das erfindungsgemäße Verfahren in Anwesenheit eines Verdünnungsmittels durchgeführt.

Beispiele für geeignete Verdünnungsmittel sind C₃-C₆-Alkohole, bevorzugt C₄-C₆-Alkohole, wie z.B. tertiäre Monoole, besonders bevorzugt tert.-Butanol, tert.-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyethan, Diethylenglycoldimethylether, Polyethylenglycoldimethylether 500, Methyltert.-butylether, Ethyl-tert.-butylether, C₁-C₄-Alkylencarbonate, insbesondere Propylencarbonat, C₃-C₆-Alkylessigsäureester, insbesondere tert.-Butylessigsäureester, Tetrahydrofuran, Toluol, 1,3-Dioxolan, Aceton, iso-Butylmethylketon, Ethylmethylketon, 1,4-Dioxan, tert.-Butylmethylether, Cyclohexan, Methylcyclohexan, Toluol, Hexan, Dimethoxymethan, 1,1-Dimethoxyethan oder Acetonitril.

Es können auch Gemische dieser Verdünnungsmittel eingesetzt werden.

Es kann vorteilhaft sein, freigesetzten Alkohol durch ein möglichst nahe am Temperaturoptimum des eingesetzten Enzyms siedendes binäres oder ternäres Heteroazeotrop abzutrennen. Der so abgetrennte Alkohol kann dann durch Phasenscheidung oder Membrandampftrennung entfernt werden.

Wahlweise können zu den organischen Verdünnungsmitteln wässrige Verdünnungsmittel zugesetzt werden, so dass - je nach organischem Verdünnungsmittel - ein- oder mehrphasige Reaktionsgemische entstehen. Beispiele für wässrige Verdünnungsmittel sind Wasser oder wässrige, verdünnte (z.B. 10 bis 100 mM) Puffer, beispielsweise mit einem pH-Wert im Bereich von etwa 6 bis 8, wie z.B. Kaliumphosphat- oder TRIS-HCl-Puffer.

Die Reaktionsgemische sind im Allgemeinen weitgehend wasserfrei, d.h. die Reaktionsgemische enthalten im Allgemeinen unter 10, bevorzugt unter 5, besonders bevorzugt unter 1 und ganz besonders bevorzugt unter 0,5 Vol-% Wasser.

Vorzugsweise werden die Reaktanden ohne Vorbehandlung (z.B. Trocknung oder Wasserdotierung) eingesetzt.

Unter einem Verdünnungsmittel versteht man im Rahmen der Erfindung ein Mittel, das die mindestens eine eingesetzte Verbindung der Formel (II) und die eingesetzte Verbindung der Formel (III) verdünnt.

In einer bevorzugten Ausführungsform der Erfindung wird das erfindungsgemäße Verfahren in Abwesenheit eines Verdünnungsmittels durchgeführt.

Bevorzugt wird das erfindungsgemäße Verfahren in Anwesenheit eines sauerstoffhaltigen Gases, z.B. in Anwesenheit von Luft oder eines Luft-Sauerstoff-Gemisches, durchgeführt. Besonders bevorzugt wird das erfindungsgemäße Verfahren in Anwesenheit von Luft durchgeführt.

Im Allgemeinen wird das erfindungsgemäße Verfahren bei Drücken im Bereich von 0 bis 1023 mbar, bevorzugt 500 bis 1018 mbar, besonders bevorzugt 800 bis 1013 mbar, ganz besonders bevorzugt bei einem Druck von 1013 mbar durchgeführt.

In einer ganz besonders bevorzugten Ausführungsform der Erfindung wird das erfindungsgemäße Verfahren bei Atmosphärendruck durchgeführt. Unter Atmosphärendruck versteht man im Rahmen der Erfindung einen Druck im Bereich von 1003 bis 1023 mbar, bevorzugt einen Druck im Bereich von 1008 bis 1018 mbar, besonders bevorzugt einen Druck von 1013 mbar.

In einer weiteren Ausführungsform der Erfindung wird das erfindungsgemäße Verfahren bei Drücken im Bereich von 0 bis 1013 mbar, bevorzugt 0 bis 500 mbar, besonders bevorzugt 0 bis 100 mbar, ganz besonders bevorzugt 0 bis 10 mbar durchgeführt.

In einer weiteren Ausführungsform der Erfindung wird das erfindungsgemäße Verfahren unter Vakuum durchgeführt. Unter Vakuum versteht man im Rahmen der Erfindung einen Druck im Bereich von 0 bis 10 mbar, bevorzugt einen Druck im Bereich von 0 bis 5 mbar, besonders bevorzugt einen Druck im Bereich von 0 bis 1 mbar.

Die Umsetzung kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren erfolgen. Solche Reaktoren sind dem Fachmann bekannt. Bevorzugt erfolgt die Umsetzung in einem Rührkesselreaktor oder in einem Festbettreaktor.

Zur Durchmischung können beliebige Verfahren eingesetzt werden. Das Reaktionsgemisch kann beispielsweise gerührt werden. Spezielle Rührvorrichtungen sind nicht erforderlich. Das Reaktionsgemisch kann beispielsweise geschüttelt werden. Spezielle Schüttelvorrichtungen sind nicht erforderlich.

Falls ein Verdünnungsmittel oder Gemische von Verdünnungsmitteln verwendet werden, können die eingesetzten Reaktanden und die gegebenenfalls eingesetzten Additive beispielsweise darin gelöst, suspendiert oder emulgiert, gegebenenfalls vorgelegt werden, und zum Start der Reaktion, sowie gegebenenfalls ein- oder mehrmals während des Reaktionsverlaufs, mit Enzym versetzt werden. Falls kein Verdünnungsmittel verwendet wird, können die eingesetzten Reaktanden und die gegebenenfalls eingesetzten Additive beispielsweise vorgelegt werden und zum Start der Reaktion, sowie gegebenenfalls ein- oder mehrmals während des Reaktionsverlaufs, mit Enzym versetzt werden. Die Temperatur kann zum Start der Reaktion auf den gewünschten Wert eingestellt und, falls gewünscht, während des Reaktionsverlaufs erhöht oder verringert werden.

Wird die Umsetzung in einem Festbettreaktor durchgeführt, so ist der Festbettreaktor bevorzugt mit immobilisiertem Enzym bestückt, wobei das Reaktionsgemisch durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Das Reaktionsgemisch kann kontinuierlich durch die Säule gepumpt werden, wobei mit der Fließgeschwindigkeit die Verweilzeit und damit der gewünschte Umsatz steuerbar sind. Es ist auch möglich, das Reaktionsgemisch im Kreislauf durch eine Säule zu pumpen, wobei freigesetzter Alkohol, z.B. unter Vakuum, gleichzeitig abdestilliert werden kann.

Freigesetzter Alkohol kann kontinuierlich oder schrittweise in an sich bekannter Weise entfernt werden, z.B. durch Destillation, Vakuum, azeotrope Entfernung, Absorption, Pervaporation oder Diffusion über Membranen.

Die Aufarbeitung der Reaktionsgemische erfolgt nach dem Fachmann bekannten Methoden, beispielsweise durch Filtration (z.B. zur Abtrennung von gegebenenfalls eingesetztem Molekularsieb) und/oder Destillation (z.B. zur Entfernung von einer gegebenenfalls im Überschuss eingesetzten Verbindung der Formel (II) wie Methylacrylat, Ethylacrylat, Methylmethacrylat oder Ethylmethacrylat). Die Produkte fallen zum Teil in Form zäher Öle an, die unter vermindertem Druck und bei mäßig erhöhter Temperatur von flüchtigen Anteilen befreit oder gereinigt werden. Sofern die Produkte als Feststoffe erhalten werden, kann die Reinigung auch durch Umkristallisieren oder Digerieren erfolgen.

Nach Beendigung der Reaktion kann das erhaltene Reaktionsgemisch ohne weitere Reinigung weiterverwendet oder gegebenenfalls in einem weiteren Schritt gereinigt werden. In der Regel werden in einem weiteren Reinigungsschritt lediglich das eingesetzte Enzym, gegebenenfalls verwendetes Verdünnungsmittel und ein gegebenenfalls vorhandener Überschuss an z.B. Methylacrylat, Ethylacrylat, Methylmethacrylat oder Ethylmethacrylat von dem erhaltenen Reaktionsgemisch abgetrennt. Eine Abtrennung des eingesetzten Enzyms erfolgt in der Regel durch Filtration, Absorption, Zentrifugation oder Dekantieren. Das abgetrennte Enzym kann anschließend für weitere Reaktionen verwendet werden. Eine Abtrennung von gegebenenfalls verwendetem Verdünnungsmittel erfolgt in der Regel durch Destillation, Rektifikation oder bei festen Reaktionsprodukten durch Filtration. Zur weiteren Reinigung der Reaktionsprodukte kann auch eine Chromatographie durchgeführt werden.

Ein weiterer Gegenstand der Erfindung ist eine Verbindung der Formel (Ia), 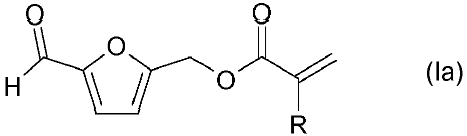 worin R n-Propyl, iso-Propyl, n-Butyl, iso-Butyl, sec-Butyl, tert-Butyl, n-Pentyl, iso-Pentyl, sec-Pentyl, tert-Pentyl, neo-Pentyl, Hexyl ist.

Die Verbindungen der Formel (I), vorzugsweise die erfindungsgemäß hergestellten Verbindungen der Formel (I), oder die erfindungsgemäßen Verbindungen der Formel (Ia) eignen sich beispielsweise als Comonomere in Dispersionen und härtbaren Zusammensetzungen.

Die HMF-Struktur verbessert die Haftungseigenschaften von z.B. Beschichtungen auf Kunststoffen, aber auch auf anderen Materialien wie Holz oder zementären Systemen.

Die Verbindungen der Formel (I), vorzugsweise die erfindungsgemäß hergestellten Verbindungen der Formel (I), oder die erfindungsgemäßen Verbindungen der Formel (Ia) finden beispielsweise Anwendung als Monomere oder Comonomere in der Herstellung von Dispersionen, die u.a. als Klebstoffe, Anstrichmittel oder Textil-, Leder- und Papierhilfsmittel eingesetzt werden.

Darüber hinaus können die Verbindungen der Formel (I), vorzugsweise die erfindungsgemäß hergestellten Verbindungen der Formel (I), oder die erfindungsgemäßen Verbindungen der Formel (Ia) als Comonomere in Polymeren Anwendung finden, die wiederum als Additiv für Brennstofföle und Schmierstoffe und insbesondere als Kaltfließverbesserer in Brennstoffölen eingesetzt werden. Eine derartige Verwendung ist beispielsweise in der europäischen Patentanmeldung EP 1 923 454 A1 offenbart.

Die Verbindungen der Formel (I), vorzugsweise die erfindungsgemäß hergestellten Verbindungen der Formel (I), oder die erfindungsgemäßen Verbindungen der Formel (Ia) finden beispielsweise auch Anwendung als Monomere oder Comonomere in der Herstellung von Dispersionen, die u.a. als Druckfarben oder Drucktinten eingesetzt werden.

Die Verbindungen der Formel (I), vorzugsweise die erfindungsgemäß hergestellten Verbindungen der Formel (I), oder die erfindungsgemäßen Verbindungen der Formel (Ia) finden beispielsweise auch Anwendung als Monomere oder Comonomere in der Herstellung von Dispersionen, die u.a. in der Kosmetik, insbesondere als Pflegeprodukte, z.B. als Hautpflegeprodukte, Haarpflegeprodukte oder Nagelpflegeprodukte, eingesetzt werden.

Die Verbindungen der Formel (I), vorzugsweise die erfindungsgemäß hergestellten Verbindungen der Formel (I), oder die erfindungsgemäßen Verbindungen der Formel (Ia) finden beispielsweise auch Anwendung als Monomere oder Comonomere in der Herstellung von Dispersionen, die u.a. für Beschichtungen im Automobilbereich, für industrielle Beschichtungen, für Beschichtungen im Gebäudebau, als Klebstoffe, z.B. druckempfindliche Klebstoffe, für Papierbeschichtungen oder als Druckfarben oder Drucktinten eingesetzt werden.

Offenbart wird die Verwendung der Verbindungen der Formel (I), vorzugsweise der erfindungsgemäß hergestellten Verbindungen der Formel (I), oder der erfindungsgemäßen Verbindungen der Formel (Ia) als Monomer oder Comonomer in der Herstellung einer Dispersion.

Die hergestellten Dispersionen können
eine oder mehrere Verbindungen der Formel (I), vorzugsweise eine oder mehrere erfindungsgemäß hergestellte Verbindungen der Formel (I), oder eine oder mehrere erfindungsgemäße Verbindungen der Formel (Ia)
und/oder
Oligomere, die unter Verwendung von einer oder mehreren Verbindungen der Formel (I), vorzugsweise einer oder mehreren erfindungsgemäß hergestellten Verbindungen der Formel (I), oder einer oder mehreren erfindungsgemäßen Verbindungen der Formel (Ia) als Monomer oder Comonomer hergestellt wurden,
und/oder
Polymere, die unter Verwendung von einer oder mehreren Verbindungen der Formel (I), vorzugsweise einer oder mehreren erfindungsgemäß hergestellten Verbindungen der Formel (I), oder einer oder mehreren erfindungsgemäßen Verbindungen der Formel (Ia) als Monomer oder Comonomer hergestellt wurden,
enthalten.

Beispielsweise enthalten die hergestellten Dispersionen eine oder mehrere Verbindungen der Formel (I), vorzugsweise eine oder mehrere erfindungsgemäß hergestellte Verbindungen der Formel (I), oder eine oder mehrere erfindungsgemäße Verbindungen der Formel (Ia).

Beispielsweise enthalten die hergestellten Dispersionen Oligomere, die unter Verwendung von einer oder mehreren Verbindungen der Formel (I), vorzugsweise einer oder mehreren erfindungsgemäß hergestellten Verbindungen der Formel (I), oder einer oder mehreren erfindungsgemäßen Verbindungen der Formel (Ia) als Monomer oder Comonomer hergestellt wurden. Ein solches Oligomer ist aus 2 bis 8 Repetiereinheiten aufgebaut.

Beispielsweise enthalten die hergestellten Dispersionen Polymere, die unter Verwendung von einer oder mehreren Verbindungen der Formel (I), vorzugsweise einer oder mehreren erfindungsgemäß hergestellten Verbindungen der Formel (I), oder einer oder mehreren erfindungsgemäßen Verbindungen der Formel (Ia) als Monomer oder Comonomer hergestellt wurden. Ein solches Polymer aus ≥ 9, z.B. ≥ 50, ≥ 100, oder ≥ 1000 Repetiereinheiten aufgebaut.

Beispielsweise weisen die hergestellten Dispersionen einen geringen Monomergehalt auf, was bedeutet, dass die hergestellten Dispersionen 0 bis 5 Gew.-%, z.B. 0 bis 2 Gew.-%, 0 bis 1 Gew.-%, oder 0 bis 0,1 Gew.-% an Verbindungen der Formel (I), vorzugsweise erfindungsgemäß hergestellten Verbindungen der Formel (I), oder erfindungsgemäßen Verbindungen der Formel (Ia) enthalten (bezogen auf die Summe der in den hergestellten Dispersionen enthaltenen Verbindungen der Formeln (I) oder (Ia); Oligomere, die unter Verwendung von Verbindungen der Formeln (I) oder (Ia) als Monomer oder Comonomer hergestellt wurden; und Polymere, die unter Verwendung von Verbindungen der Formeln (I) oder (Ia) als Monomer oder Comonomer hergestellt wurden).

Beispielsweise weisen die hergestellten Dispersionen einen geringen Oligomergehalt auf, was bedeutet, dass die hergestellten Dispersionen 0 bis 5 Gew.-%, z.B. 0 bis 2 Gew.-%, 0 bis 1 Gew.-%, oder 0 bis 0,1 Gew.-% an Oligomeren, die unter Verwendung von einer oder mehreren Verbindungen der Formel (I), vorzugsweise einer oder mehreren erfindungsgemäß hergestellten Verbindungen der Formel (I), oder einer oder mehreren erfindungsgemäßen Verbindungen der Formel (Ia) als Monomer oder Comonomer hergestellt wurden, enthalten (bezogen auf die Summe der in den hergestellten Dispersionen enthaltenen Verbindungen der Formeln (I) oder (Ia); Oligomere, die unter Verwendung von Verbindungen der Formeln (I) oder (Ia) als Monomer oder Comonomer hergestellt wurden; und Polymere, die unter Verwendung von Verbindungen der Formeln (I) oder (Ia) als Monomer oder Comonomer hergestellt wurden).

Beispielsweise weisen die hergestellten Dispersionen einen hohen Polymergehalt auf, was bedeutet, dass die hergestellten Dispersionen 90 bis 100 Gew.-%, z.B. 96 bis 100 Gew.-%, 98 bis 100 Gew.-%, oder 99,8 bis 100 Gew.-% an Polymeren, die unter Verwendung von einer oder mehreren Verbindungen der Formel (I), vorzugsweise einer oder mehreren erfindungsgemäß hergestellten Verbindungen der Formel (I), oder einer oder mehreren erfindungsgemäßen Verbindungen der Formel (Ia) als Monomer oder Comonomer hergestellt wurden, enthalten (bezogen auf die Summe der in den hergestellten Dispersionen enthaltenen Verbindungen der Formeln (I) oder (Ia); Oligomere, die unter Verwendung von Verbindungen der Formeln (I) oder (Ia) als Monomer oder Comonomer hergestellt wurden; und Polymere, die unter Verwendung von Verbindungen der Formeln (I) oder (Ia) als Monomer oder Comonomer hergestellt wurden).

Beispielsweise weisen die hergestellten Dispersionen einen geringen Monomergehalt, einen geringen Oligomergehalt und einen hohen Polymergehalt auf. Die Begriffe "geringer Monomergehalt", "geringer Oligomergehalt" und "hoher Polymergehalt" sind vorstehend definiert.

Beispielsweise werden die hergestellten Dispersionen als Klebstoffe, Anstrichmittel, Textil-, Leder- oder Papierhilfsmittel oder als Additiv für Brennstofföle und Schmierstoffe eingesetzt.

Offenbart ist demnach die Verwendung der hergestellten Dispersionen, wobei die Dispersionen als Klebstoffe, Anstrichmittel, Textil-, Leder- oder Papierhilfsmittel oder als Additiv für Brennstofföle und Schmierstoffe eingesetzt werden.

Beispielsweise werden die hergestellten Dispersionen als Druckfarben oder Drucktinten eingesetzt.

Offenbart ist demnach die Verwendung der hergestellten Dispersionen, wobei die Dispersionen als Druckfarben oder Drucktinten eingesetzt werden.

Beispielsweise werden die hergestellten Dispersionen in der Kosmetik, insbesondere als Pflegeprodukte, z.B. als Hautpflegeprodukte, Haarpflegeprodukte oder Nagelpflegeprodukte, eingesetzt.

Offenbart ist demnach die Verwendung der hergestellten Dispersionen, wobei die Dispersionen in der Kosmetik, insbesondere als Pflegeprodukte, z.B. als Hautpflegeprodukte, Haarpflegeprodukte oder Nagelpflegeprodukte, eingesetzt werden.

Beispielsweise werden die hergestellten Dispersionen für Beschichtungen im Automobilbereich, für industrielle Beschichtungen, für Beschichtungen im Gebäudebau, als Klebstoffe, z.B. druckempfindliche Klebstoffe, für Papierbeschichtungen oder als Druckfarben oder Drucktinten eingesetzt.

Offenbart ist demnach die Verwendung der hergestellten Dispersionen, wobei die Dispersionen für Beschichtungen im Automobilbereich, für industrielle Beschichtungen, für Beschichtungen im Gebäudebau, als Klebstoffe, z.B. druckempfindliche Klebstoffe, für Papierbeschichtungen oder als Druckfarben oder Drucktinten eingesetzt werden.

Beispielsweise werden die hergestellten Dispersionen für Beschichtungen, insbesondere Beschichtungen im Automobilbereich, industrielle Beschichtungen oder Beschichtungen im Gebäudebau, als Klebstoffe, als Druckfarben oder Drucktinten oder in der Kosmetik eingesetzt.

Offenbart ist demnach die Verwendung der hergestellten Dispersionen, wobei die Dispersionen für Beschichtungen, insbesondere Beschichtungen im Automobilbereich, industrielle Beschichtungen oder Beschichtungen im Gebäudebau, als Klebstoffe, als Druckfarben oder Drucktinten oder in der Kosmetik eingesetzt werden.

Die hergestellten Dispersionen können z.B. auch für Beschichtungen im Innen- oder Außenbereich, z.B. von Wänden, Böden oder Decken, insbesondere auch als Wandfarben, Farben für Böden oder Farben für Decken, eingesetzt werden.

Die hergestellten Dispersionen können z.B. auch für Beschichtungen von Mauerwerken im Innen- oder Außenbereich eingesetzt werden.

Die hergestellten Dispersionen können z.B. auch für Straßenmarkierungen eingesetzt werden.

Ein Vorteil der Verbindungen der Formel (I), vorzugsweise der erfindungsgemäß hergestellten Verbindungen der Formel (I), oder der erfindungsgemäßen Verbindungen der Formel (Ia) ist, dass sie wegen ihrer geringen Farbzahl in Lackanwendungen und dort insbesondere in Klarlacken vorteilhaft eingesetzt werden können, da sie durch ihre geringe Eigenfärbung eine verringerte Färbung der Beschichtungen gegenüber nach konventionellen Verfahren hergestellten (Meth)acrylaten bewirken.

Ferner können Beschichtungen mit den Verbindungen der Formel (I), vorzugsweise den erfindungsgemäß hergestellten Verbindungen der Formel (I), oder den erfindungsgemäßen Verbindungen der Formel (Ia) sehr hohe Kratzfestigkeiten, Härten, Chemikalienbeständigkeiten, Elastizität und Haftung, sowohl auf hydrophilen als auch auf hydrophoben Substraten aufweisen.

Die Verbindungen der Formel (I), vorzugsweise die erfindungsgemäß hergestellten Verbindungen der Formel (I), oder die erfindungsgemäßen Verbindungen der Formel (Ia) können vorteilhaft als Monomere oder Comonomere in Poly(meth)acrylaten oder als Reaktivverdünner in thermisch-, strahlungs- und/oder Dual-Cure-härtbaren Poly(meth)acrylaten eingesetzt werden. Derartige Poly(meth)acrylate sind beispielsweise als Bindemittel in thermisch-, strahlungs- oder Dual-Cure-härtbaren Beschichtungsmitteln sowie in Klebstoffen, z. B. Acrylatklebstoffen, geeignet sowie in Dichtungsmassen.

Offenbart ist daher die Verwendung der Verbindungen der Formel (I), vorzugsweise der erfindungsgemäß hergestellten Verbindungen der Formel (I), oder der erfindungsgemäßen Verbindungen der Formel (Ia) als Reaktivverdünner oder Bindemittel in Strahlen- oder Dual-Cure-härtbaren Beschichtungsmassen, beispielsweise in Deckbeschichtungen, z.B. in transparenten Klarlacken. Selbstverständlich können die Verbindungen der Formel (I), vorzugsweise die erfindungsgemäß hergestellten Verbindungen der Formel (I), oder die erfindungsgemäßen Verbindungen der Formel (Ia) auch als Monomere in Polymerisationen, gegebenenfalls zusammen mit anderen polymerisierbaren Monomeren, wie z. B. (Meth)acrylsäure, (Meth)acrylsäureester, Styrol, Butadien, Acrylnitril, Vinylacetat, N-Vinylpyrrolidon, 4-Hydroxybutylvinylether oder N-Vinylformamid, verwendet werden.

Unter "Dual-Cure" ist zu verstehen, dass die Beschichtungsmassen thermisch und mit aktinischer Strahlung härtbar sind. Im Rahmen der vorliegenden Erfindung ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen. Strahlenhärtbare Bindemittel sind solche, die mittels aktinischer Strahlung wie vorstehend definiert, insbesondere mittels UV-Strahlung, härtbar sind.

Offenbart sind Lackformulierungen, enthaltend die Verbindungen der Formel (I), vorzugsweise die erfindungsgemäß hergestellten Verbindungen der Formel (I), oder die erfindungsgemäßen Verbindungen der Formel (Ia). Dabei können die Verbindungen der Formel (I), vorzugsweise die erfindungsgemäß hergestellten Verbindungen der Formel (I), oder die erfindungsgemäßen Verbindungen der Formel (Ia) sowohl in Basislacken als auch in Decklacken eingesetzt werden. Aufgrund ihrer besonderen Eigenschaften, insbesondere ihrer geringen Farbzahl, ist ihr Einsatz in Deckbeschichtungen und einer strahlengehärteten Klarlackbeschichtungen offenbart.

Neben den Verbindungen der Formel (I), vorzugsweise den erfindungsgemäß hergestellten Verbindungen der Formel (I), oder den erfindungsgemäßen Verbindungen der Formel (la) kann eine strahlungshärtbare Masse noch folgende Komponenten enthalten:
(G) mindestens eine polymerisierbare Verbindung mit mehreren copolymerisierbaren, ethylenisch ungesättigten Gruppen,
(H) gegebenenfalls Reaktivverdünner,
(P) gegebenenfalls Photoinitiatoren sowie
(J) gegebenenfalls weitere lacktypische Additive.

Als Verbindungen (G) kommen strahlungshärtbare, radikalisch polymerisierbare Verbindungen mit mehreren, d. h. mindestens zwei, copolymerisierbaren, ethylenisch ungesättigten Gruppen in Betracht.

Als Reaktivverdünner (Verbindungen (H)) kommen strahlungshärtbare, radikalisch oder kationisch polymerisierbare Verbindungen mit nur einer ethylenisch ungesättigten, copolymerisierbaren Gruppe in Betracht.

Als Photoinitiatoren (P) können dem Fachmann bekannte Photoinitiatoren verwendet werden, z. B. die in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SI-TA Technology Ltd, London, genannten.

Als weitere lacktypische Additive (J) können beispielsweise Antioxidantien, Oxidationsinhibitoren, Stabilisatoren, Aktivatoren (Beschleuniger), Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Beispiele für die genannten Verbindungsklassen (G), (H), (P) und (J) sind in WO 2006/005491 und in DE 10 2005 037 430 offenbart.

Typische Zusammensetzungen für strahlungshärtbare Massen sind beispielsweise
(I) oder (Ia) 20 - 100 Gew.-%, z.B. 40 - 90, 50 - 90, oder 60 - 80 Gew.-%,
(G) 0 - 60 Gew.-%, z.B. 5 - 50, 10 - 40, oder 10 - 30 Gew.-%,
(H) 0 - 50 Gew.-%, z.B. 5 - 40, 6 - 30, oder 10 - 30 Gew.-%,
(P) 0 - 20 Gew.-%, z.B. 0,5 - 15, 1 - 10, oder 2 - 5 Gew.-% sowie
(J) 0 - 50 Gew.-%, z.B. 2 - 40, 3 - 30, oder 5 - 20 Gew.-%,
mit der Maßgabe, dass (I) oder (Ia), (G), (H), (P) und (J) zusammen 100 Gew.-% ergeben.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Beschichtungsmasse, gegebenenfalls unter Erhitzen, entfernt. Dieser Vorgang kann, falls gewünscht, ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m² und vorzugsweise 10 bis 200 g/m².

Die Verbindungen der Formel (I), vorzugsweise die erfindungsgemäß hergestellten Verbindungen der Formel (I), oder die erfindungsgemäßen Verbindungen der Formel (la) können aufgrund ihrer geringeren Färbung vorteilhaft auch in einer thermisch induzierten (radikalischen) (Co)polymerisation eingesetzt werden.

Als Monomere, mit denen die Verbindungen der Formel (I), vorzugsweise die erfindungsgemäß hergestellten Verbindungen der Formel (I), oder die erfindungsgemäßen Verbindungen der Formel (Ia) beispielsweise copolymerisiert werden können, seien z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen und aliphatischen Kohlenwasserstoffen mit 2 bis 8 C-Atomen und 1 oder 2 Doppelbindungen genannt.

Als (Meth)acrylsäurealkylester geeignet sind solche mit einem C₁-C₁₀-Alkylrest, wie Methylmethacrylat, Methylacrylat, n-Butylacrylat, Ethylacrylat und verzweigte Alkylderivate wie 2-Ethylhexylacrylat.

Insbesondere sind auch Mischungen der (Meth)acrylsäurealkylester geeignet.

Vinylester von Carbonsäuren mit 1 bis 20 C-Atomen sind z. B. Vinyllaurat, Vinylstearat, Vinylpropionat und Vinylacetat.

Als vinylaromatische Verbindungen kommen z. B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z. B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Als Monomere, mit denen die Verbindungen der Formel (I), vorzugsweise die erfindungsgemäß hergestellten Verbindungen der Formel (I), oder die erfindungsgemäßen Verbindungen der Formel (la) beispielsweise copolymerisiert werden können, seien Vinylmonomere wie z.B. 1,3-Butadien, Isopren, Styrol, substituierte Styrole, Divinylbenzol, heterocyclische Vinylverbindungen oder Vinylhalogenide; Vinylester wie z.B. Vinylformiat, Vinylacetat, Vinylpropionat, Vinylversatat oder Vinyllaurat; Vinylether wie z.B. Methylvinylether, Ethylvinylether, Vinyl-2-methoxy-ethylether oder Vinyl-2-chlorethylether; (Meth)acrylester mit C₁-C₂₄-Alkoholen wie z.B. Methyl(meth)acrylat, Ethyl(meth)acrylat, Butyl(meth)acrylat, 2-Ethylhexyl(meth)acrylat, Isopropyl(meth)acrylat, Hydroxyethyl(meth)acrylat, Hydroxypropyl(meth)acrylat, n- oder Isopropyl(meth)acrylat, Amyl(meth)acrylat, Isoamyl(meth)acrylat, tert.-Amyl(meth)acrylat, Hexyl(meth)acrylat, Cyclohexyl(meth)acrylat, Octyl(meth)acrylat, Decyl(meth)acrylat, Lauryl(meth)acrylat, Myristyl(meth)acrylat, Cetyl(meth)acrylat, Stearyl(meth)acrylat; (Meth)acrylester von Ether-Alkoholen wie z.B. Ethylenglykolmonomethylether(meth)acrylat, sowie Di(meth)acrylate von C₁-C₆-Diolen wie z.B. 1,6-Hexandioldi(meth)acrylat, oder (Meth)acrylsäure bzw. andere, vinylisch ungesättigte Carbonsäuren, Carbonsäureamide oder - nitrile genannt.

Eine häufige, aber nicht die einzige Methode zur Herstellung solcher (Co)Polymerisate ist die radikalische oder ionische (Co)Polymerisation in einem Lösungs- oder Verdünnungsmittel.

Die radikalische (Co)Polymerisation solcher Monomere erfolgt beispielsweise in wässriger Lösung in Gegenwart von Polymerisationsinitiatoren, die unter Polymerisationsbedingungen in Radikale zerfallen, beispielsweise Peroxodisulfate, H₂O₂-Redoxsysteme oder Hydroperoxide, wie z. B. tert.-Butylhydroperoxid oder Cumolhydroperoxid. Die (Co)Polymerisation kann in einem weiten Temperaturbereich, gegebenenfalls unter vermindertem oder auch unter erhöhtem Druck, in der Regel bei Temperaturen bis zu 100 °C durchgeführt werden. Der pH-Wert des Reaktionsgemisches wird gewöhnlich auf einen Wert im Bereich von 4 bis 10 eingestellt.

Die (Co)Polymerisation kann aber auch in anderer, dem Fachmann an sich bekannter Weise kontinuierlich oder diskontinuierlich durchgeführt werden, z. B. als Lösungs-, Fällungs-, Wasserin-ÖI-Emulsions-, inverse Emulsions-, Suspensions- oder umgekehrte Suspensionspolymerisation.

Dabei wird/werden das Monomer/die Monomere unter Verwendung radikalischer Polymerisationsinitiatoren, z. B. in Radikale zerfallende Azoverbindungen, wie 2,2'-Azo-bis(isobutyronitril), 2,2'-Azobis-(2-amidinopropan)-hydrochlorid oder 4,4'-Azo-bis-(4'-cyanpentansäure) oder Dialkylperoxide, wie Di-tert.-amylperoxid, Arylalkylperoxide, wie tert.-Butylcumylperoxid, Alkylacylperoxide, wie tert.-Butylperoxy-2-ethylhexanoat, Peroxydicarbonate, wie Di-(4-tert.-Butylcyclohexyl)peroxydicarbonat oder Hydroperoxide (co)polymerisiert.

Weitere Beispiele für geeignete Polymerisationsinitiatoren sind Peroxide, Hydroperoxide, Wasserstoffperoxid, Persulfate, Azoverbindungen oder die sogenannten Redoxinitiatoren.

Weitere Beispiele für geeignete Polymerisationsinitiatoren sind Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butyl-per-2-ethyl-hexanoat, tert.-Butylperisononanoat, tert.-Butylpermaleat, tert.-Butylperbenzoat, Di-(2-ethylhexyl)-peroxydicarbonat, Dicyclohexylperoxydicarbonat, Di-(4-tert.-butylcyclohexyl)peroxy-dicarbonat, Dimyristilperoxydicarbonat, Diacetylperoxydicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid oder tert.-Amylpemeodekanoat.

Beispiele für geeignete Polymerisationsinitiatoren sind Azoverbindungen, beispielsweise 2,2'-Azobisisobutyronitril, 2,2'-Azobis(2,4-dimethylvaleronitril) und 2,2'-Azobis(4-methoxy-2,4-dimethylvaleronitril).

Die genannten Verbindungen werden meist in Form wässriger Lösungen oder wässriger Emulsionen eingesetzt, wobei die untere Konzentration durch die in der (Co)Polymerisation vertretbare Wassermenge und die obere Konzentration durch die Löslichkeit der betreffenden Verbindung in Wasser bestimmt ist.

Als Lösungs- oder Verdünnungsmittel können z. B. Wasser, Alkohole, wie Methanol, Ethanol, n- oder iso-Propanol, n- oder iso-Butanol, oder Ketone, wie Aceton, Ethylmethylketon, Diethylketon oder iso-Butylmethylketon dienen. Geeignet sind unpolare Lösungsmittel, wie beispielsweise Xylol und dessen Isomerengemische, Shellsol® A und Solventnaphtha.

Beispielsweise werden die Monomere vorgemischt und Initiator mit gegebenenfalls weiteren Zusätzen gelöst in Lösungsmittel zugegeben. Ein Verfahren ist beschrieben in WO 2001/23484 und dort besonders auf Seite 10, Zeile 3 bis Zeile 24.

Gegebenenfalls kann die (Co)Polymerisation in Gegenwart von Polymerisationsreglern, wie beispielsweise Hydroxylammoniumsalze, chlorierte Kohlenwasserstoffe und Thioverbindungen, wie z.B. tert.-Butylmercaptan, Thioglycolsäureethylacrylester, Mercaptoethanol, Mercaptopropyltrimethoxysilan, Dodecylmercaptan, tert.-Dodecylmercaptan oder Alkalimetallhypophosphite, durchgeführt werden. Bei der (Co)Polymerisation können diese Regler z. B. in Mengen von 0 bis 0,8 Gew.-Teile, bezogen auf 100 Gew.-Teile der zu (co)polymerisierenden Monomeren, eingesetzt werden, durch die die Molmasse des entstehenden (Co)Polymers verringert wird.

Bei der Emulsionspolymerisation können Dispergiermittel, ionische und/oder nicht-ionische Emulgatoren und/oder Schutzkolloide bzw. Stabilisatoren als grenzflächenaktive Verbindungen verwendet werden. Als solche kommen sowohl die zur Durchführung von Emulsionspolymerisationen üblicherweise eingesetzten Schutzkolloide als auch Emulgatoren in Betracht.

Geeignete Schutzkolloide sind beispielsweise Polyvinylalkohole, Cellulosederivate oder Vinylpyrrolidon enthaltende Copolymerisate. Eine ausführliche Beschreibung weiterer geeigneter Schutzkolloide findet sich in Houben-Weyl, Methoden der organischen Chemie, Band XIV/1, makromolekulare Stoffe, Georg-Thieme-Verlag, Stuttgart, 1969, S. 411 bis 420. Selbstverständlich können auch Gemische aus Emulgatoren und/oder Schutzkolloiden verwendet werden.

Beispielsweise werden als Dispergiermittel ausschließlich Emulgatoren eingesetzt, deren relative Molekulargewichte im Unterschied zu den Schutzkolloiden üblicherweise unter 1.000 liegen. Sie können sowohl anionischer, kationischer oder nicht-ionischer Natur sein. Selbstverständlich müssen im Falle der Verwendung von Gemischen grenzflächenaktiver Substanzen die Einzelkomponenten miteinander verträglich sein, was im Zweifelsfall an Hand weniger Vorversuche überprüft werden kann. Im Allgemeinen sind anionische Emulgatoren untereinander und mit nicht-ionischen Emulgatoren verträglich.

Desgleichen gilt auch für kationische Emulgatoren, während anionische und kationische Emulgatoren meistens miteinander unverträglich sind. Gebräuchliche Emulgatoren sind z.B. ethoxylierte Mono-, Di- und Tri-Alkylphenole (EO-Grad: 3 bis 100, Alkylrest: C₄ bis C₁₂), ethoxylierte Fettalkohole (EO-Grad: 3 bis 100, Alkylrest: C₈ bis C₁₈), sowie Alkali- und Ammoniumsalze von Alkylsulfaten (Alkylrest: C₈ bis C₁₆) von Schwefelsäurehalbestern ethoxylierter Alkylphenole (EO-Grad: 3 bis 100, Alkylrest: C₄ bis C₁₂), von Alkylsulfonsäuren (Alkylrest: C₁₂ bis C₁₈) und von Alkylacrylsulfonsäuren (Alkylrest: C₉ bis C₁₈). Weitere geeignete Emulgatoren wie Sulfobernsteinsäureester finden sich in Houben-Weyl, Methoden der organischen Chemie, Band XIV/1, Makromolekulare Stoffe, Georg-Thieme Verlag, Stuttgart, 1961, Seiten 192 bis 208.

In der Regel beträgt die Menge an eingesetztem Dispergiermittel 0,5 bis 6 Gew.-%, z.B. 1 bis 3 Gew.-% bezogen auf die radikalisch zu polymerisierenden Monomeren.

Beispiele für (meth)acrylathaltige Dispersionen sind n-Butylacrylat/Acrylnitril -Dispersionen, die als Klebstoffe Anwendung finden, sowie n-Butylacrylat/Butadien/Styrol-Dispersionen.

Die Polymerdispersionen, in denen die Verbindungen der Formel (I), vorzugsweise die erfindungsgemäß hergestellten Verbindungen der Formel (I), oder die erfindungsgemäßen Verbindungen der Formel (la) verwendet werden, können zusätzlich chemisch und/oder physikalisch desodoriert werden.

Eine chemische Desodorierung kann beispielsweise wie von P.H.H. Araújo, C. Sayer, J. G. R. Poco, R. Giudici in Polymer Engineering and Science, 2002 (42), 1442-1468 beschrieben oder in EP 1 375 530 B1 offenbart durchgeführt werden.

Die mit den Verbindungen der Formel (I), vorzugsweise den erfindungsgemäß hergestellten Verbindungen der Formel (I), oder den erfindungsgemäßen Verbindungen der Formel (la) erhältlichen Copolymerisate weisen in der Regel eine geringere Farbzahl auf, was im Lackbereich vorteilhaft ist. Die beschriebenen Copolymerisate lassen sich dann in an sich bekannter Weise, beispielsweise mit Aminoplasten, wie z. B. Melamin, zu vernetzten Lackharzen umsetzen, wie es beispielsweise in EP 0 738 740 oder EP 0 675 141 beschrieben ist.

Es eignen sich die Beschichtungsmassen als oder in Außenbeschichtungen also solche Anwendungen, die dem Tageslicht ausgesetzt sind, z.B. von Gebäuden oder Gebäudeteilen, Innenbeschichtungen, Straßenmarkierungen, Beschichtungen auf Fahrzeugen und Flugzeugen. Insbesondere können die Beschichtungen auch als Holz-, Papier- oder Kunststoffbeschichtungen, beispielsweise für Parkett oder Möbel, eingesetzt werden.

Offenbart ist die Verwendung der Verbindungen der Formel (I), vorzugsweise der erfindungsgemäß hergestellten Verbindungen der Formel (I), oder der erfindungsgemäßen Verbindungen der Formel (la) als Vorprodukt für Glanzzusätze in der Galvanotechnik. Ihre gegenüber konventionell erhältlichen Produkten verringerte Farbzahl macht sie für diese Anwendung außerordentlich geeignet.

Offenbart ist die Verwendung der Verbindungen der Formel (I), vorzugsweise der erfindungsgemäß hergestellten Verbindungen der Formel (I), oder der erfindungsgemäßen Verbindungen der Formel (la) als Monomer oder Comonomer in Poly(meth)acrylaten oder als Reaktivverdünner in thermisch-, strahlungs- und/oder Dual-Cure-härtbaren Poly(meth)acrylaten, insbesondere in Dual-Cure-härtbaren Beschichtungsmassen, oder als Vorprodukt für Glanzzusätze in der Galvanotechnik.

Offenbart ist die Verwendung der Verbindungen der Formel (I), vorzugsweise der erfindungsgemäß hergestellten Verbindungen der Formel (I), oder der erfindungsgemäßen Verbindungen der Formel (la) als Reaktivverdünner in thermisch-, strahlungs- und/oder Dual-Cure-härtbaren Poly(meth)acrylaten, insbesondere in Dual-Cure-härtbaren Beschichtungsmassen.

Die Verbindungen der Formel (I), vorzugsweise die erfindungsgemäß hergestellten Verbindungen der Formel (I), oder die erfindungsgemäßen Verbindungen der Formel (la) eignen sich auch als Comonomere in nachvernetzbaren Systemen.

Nachvernetzbare Systeme sind z.B. in Iranian Polymer Journal 2008, 17 (7), 555-564 und in Progress in Polymer Science 2011, 36, 191-217 beschrieben.

Offenbart ist die Verwendung der Verbindungen der Formel (I), vorzugsweise der erfindungsgemäß hergestellten Verbindungen der Formel (I), oder der erfindungsgemäßen Verbindungen der Formel (la) zur Herstellung eines vernetzbaren Copolymers. Beispielsweise eignen sich die Verbindungen der Formel (I), vorzugsweise die erfindungsgemäß hergestellten Verbindungen der Formel (I), oder die erfindungsgemäßen Verbindungen der Formel (la) in Kombination mit vernetzungsaktiven Comonomeren zum Einsatz in selbstvernetzenden Harzen. Geeignete vernetzungsaktive Comonomere zum Einsatz in selbstvernetzenden Harzen sind Comonomere, deren funktionelle Seitengruppe mit den erfindungsgemäßen Aldehydgruppen enthaltenden Monomeren reagieren können, wie z.B. Amine, Hydrazine oder Oximgeblockte Isocyanate. Solche Comonomere sind z.B. in EP 2246403 oder in DE 4237030 beschrieben.

Die Verbindungen der Formel (I), vorzugsweise die erfindungsgemäß hergestellten Verbindungen der Formel (I), oder die erfindungsgemäßen Verbindungen der Formel (la) eignen sich auch zum Einsatz in Harzen, bei denen die Vernetzungsfunktion nicht in die Polymerkomponente selbst eingebaut ist, sondern ein separater Vernetzer zugesetzt wird. Typischerweise werden hier z.B. Amine, Diamine, Triamine, Hydroxylamine, Oxime, Oximether, Oxyamine, Dihydrazine, Dihydrazide, Trihydrazide oder Polyhydrazide eingesetzt. Weitere geeignete Vernetzer sind z.B. in WO 2006/086322 beschrieben.

Die Einsatzmenge der Verbindungen der Formel (I), vorzugsweise der erfindungsgemäß hergestellten Verbindungen der Formel (I), oder der erfindungsgemäßen Verbindungen der Formel (Ia) in den Copolymeren beträgt im Allgemeinen 0,2 bis 35 Gew.-%, z.B. 0,5 bis 20 Gew.-%, oder 1 bis 10 Gew.-%. Die Einsatzmenge der jeweils vernetzungsaktiven Comonomere kann darauf molar abgestimmt werden. Dies gilt auch für die Einsatzmenge der separaten Vernetzter.

Die vernetzbaren Systeme finden beispielsweise Anwendung in der Herstellung von Beschichtungen, Klebstoffen und Filmen für poröse und nicht-poröse Substrate wie Papier, nichtgewebte Materialien, Textilien, Leder, Holz, Beton, Mauerwerk, Metalle mit oder ohne Grundierung, Kunststoffe (z.B. Polypropylen, Polyester, Polyurethane), Baumaterialien, Gegenstände aus Polymeren, Schutzausrüstungen.

Die vernetzbaren Systeme finden beispielsweise auch Anwendung in der Herstellung von Fasermaterialen, Filmen, Platten, Composites, Tinten, Druckbindern, Flockstoffen, Klebstoffen, Pflegeprodukten, wie z.B. Hautpflegeprodukten, Haarpflegeprodukten oder Nagelpflegeprodukten.

Die vernetzbaren Systeme finden beispielsweise auch Anwendung in der Herstellung von kratzfesten Schutzschichten für den inneren oder äußeren Gebrauch, wie z.B. Plastikbeschichtungen für Fahrzeuge, Elektrogeräte oder Holzböden.

Die vernetzbaren Systeme finden beispielsweise auch Anwendung in der Beschichtung oder Imprägnierung von Teppichen oder Textilien, die für Kleidung, Polstermöbel, Zelte, Markisen und dergleichen verwendet werden können. Geeignete Textilien umfassen Stoffe, Garne oder Mischtextilien, ganz gleich, ob gewebt oder nicht gewebt oder gestrickt, und ob sie natürlich, synthetisch oder regeneriert sind. Beispiele für geeignete Textilien umfassen Zelluloseacetat, Acryl, Wolle, Baumwolle, Jute, Leinen, Polyester, Polyamide, regenerierte Cellulose (Rayon) und dergleichen.

Die Erfindung wird durch die nachfolgenden Beispiele näher erläutert.

### Beispiele

Das in den Synthesebeispielen verwendete 5-(Hydroxymethyl)-furfural (HMF) wurde von Aldrich (CAS: 67-47-0) kommerziell bezogen.

Das in den Synthesebeispielen verwendete Methylacrylat sowie Methylmethacrylat wurde von BASF bezogen.

Das in den Synthesebeispielen verwendete Enzym BASF Novozym® 435 wurde von BASF bezogen.

Der in den Synthesebeispielen verwendete Begriff HMF-Acrylat steht für die nachfolgend abgebildete Verbindung:

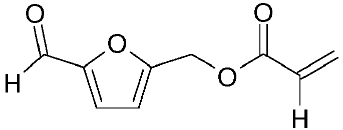

Der in den Synthesebeispielen verwendete Begriff HMF-Methacrylat steht für die nachfolgend abgebildete Verbindung:

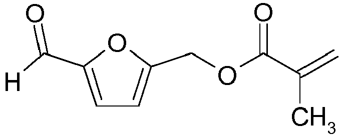

Der in den Synthesebeispielen verwendete Begriff MEHQ steht für "mono methyl ether of hydroquinone" bzw. Hydrochinonmonomethylether. Ein Synonym hierfür ist para-Methoxyphenol (PMP).

### Gaschromatographie:

Die gaschromatische Beobachtung des Reaktionsfortschritts erfolgte nach folgender Methode:
Messgerät: Agilent 6890N
Säule: RTX-200-MS _{Länge} = 30m, øᵢₙₙₑₙ = 0,32mm, ø_{außen} = 0,45 mm, Filmdicke 0,5 µm, Fa. Restec Best.Nr.: 15639
Fluss: 1,0mL/min bei 5,7PSI (gemessen bei Ofentemp. 80°C)
Split: 1:50, Splitflow: 50mL/min, Septum purge 3,0mL/min (gemessen bei Ofentemp. 80°C)
Trägergas: Stickstoff
Injektor: Split/Splitless mit siltec-deactivated Liner (Fa. Restec # 20782-213.5)
Injektortemperatur: 280°C
Injektionsvolumen: 1µL
Detektor: FID mit 300mL/min Luft, 30mL/min Wasserstoff und 30mL/min Make-Up-Gas (Stickstoff)
Detektortemperatur: 320°C

**Temperaturprogramm:**

| | |
|---|---|
| Start: | 60°C |
| Verweildauer 1: | 5 min |
| Temperaturrampe 1: | 15°C/min |
| Endtemperatur 1: | 310°C |
| Verweildauer 2: | 10 min |
| Gesamtlaufzeit: | 31,7 min |

Messungen und Ergebnisse: verdünnte Proben nach Flächen % ohne Lösungsmittel und Acrylat
Auswertung: Empower-3-Software Service Release 1 (Fa. Waters)

### Beispiel 1:

In einer 25 mL Schottflasche wurde HMF (1 g, 0,0079 mol) in Methylacrylat (6,83 g, 0,079 mol) gelöst. Zu diesem Ansatz wurden Molsieb (2,5 g, 5 Angstroem) sowie eine Spatelspitze MEHQ gegeben. Der Ansatz wurde mit dem Enzym BASF Novozym® 435 (0,075 g, 7,5 Gew.-%) versetzt und bei einer Reaktionstemperatur von 40 °C im Wasserbad geschüttelt. Der Reaktionsfortschritt wurde via Gaschromatographie beobachtet:

| Eintrag | Zeit [h] | Edukt: HMF (Retentionszeit: 14,1 min) | Produkt: HMF-Acrylat (Retentionszeit: 15,5 min) |
|---|---|---|---|
| 1 | 2 | 91,26 | 8,74 |
| 2 | 4 | 88,28 | 11,72 |
| 3 | 24 | 64,4 | 35,6 |
| 4 | 48 | 23,4 | 76,6 |

Nach 48 h wurde ein Umsatz (von HMF zu HMF-Acrylat) von 76,6 % nachgewiesen. Die Reaktion verlief außerordentlich selektiv, ohne Bildung von Nebenprodukten sowie ohne Färbung des Reaktionsansatzes. Nach filtrativer Abtrennung des Molsiebes ließ sich der Ansatz im Vakuum problemlos einengen (Entfernung des flüchtigen Methylacrylats). Der Reaktionsrückstand wurde farblos erhalten.

Die Identität des Produkts wurde via GC-MS (Masse _{theoretisch}: 180.6 (C₉H₈O₄); Masse _{gefunden}: 180) sowie 1H-NMR nachgewiesen.

### Beispiel 2:

In einer 25 mL Schottflasche wurde HMF (1 g, 0,0079 mol) in Methylmethacrylat (7,9 g, 0,079 mol) gelöst. Zu diesem Ansatz wurden Molsieb (2,5 g, 5 Angstroem) sowie eine Spatelspitze MEHQ gegeben. Der Ansatz wurde mit dem Enzym BASF Novozym® 435 (0,075 g, 7,5 Gew.- %) versetzt und bei einer Reaktionstemperatur von 40 °C im Wasserbad geschüttelt. Der Reaktionsfortschritt wurde via Gaschromatographie beobachtet. Nach 48 h wurde ein Umsatz (von HMF zu HMF-Methacrylat) von 6 % nachgewiesen.

## Patentansprüche

1. Verfahren zur Herstellung einer Verbindung der Formel (I), 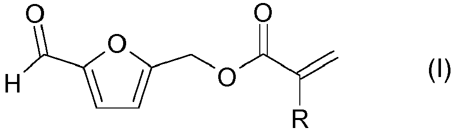 worin R H oder C₁-C₆-Alkyl ist,
durch Umsetzung von mindestens einer Verbindung der Formel (II) 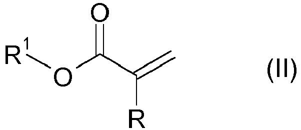
worin R die gleiche Bedeutung wie in der Formel (I) hat und
worin R¹ H, C₁-C₁₂-Alkyl oder C₃-C₁₂-Cycloalkyl ist,
mit einer Verbindung der Formel (III) 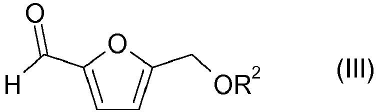
worin R² H oder C(O)R³ ist,
wobei R³ H oder C₁-C₁₂-Alkyl ist,
in Gegenwart von mindestens einem zur Umesterung geeigneten Enzym, wobei eine Lipase (EC 3.1.1.-) als Enzym eingesetzt wird.

2. Verfahren gemäß Anspruch 1, wobei R H oder CH₃ ist.

3. Verfahren gemäß Anspruch 1 oder 2, wobei R H ist.

4. Verfahren gemäß Anspruch 1 oder 2, wobei R CH₃ ist.

5. Verfahren gemäß einem der Ansprüche 1 bis 4, wobei R¹ CH₃ oder CH₂CH₃ ist.

6. Verfahren gemäß einem der Ansprüche 1 bis 5, wobei R² H ist.

7. Verfahren gemäß einem der Ansprüche 1 bis 6, wobei die mindestens eine Verbindung der Formel (II) und die Verbindung der Formel (III) in einem Molverhältnis von 5:1 bis 15:1 eingesetzt werden.

8. Verfahren gemäß einem der Ansprüche 1 bis 7, wobei das Verfahren bei Temperaturen im Bereich von 20 bis 60 °C durchgeführt wird.

9. Verbindung der Formel (la), 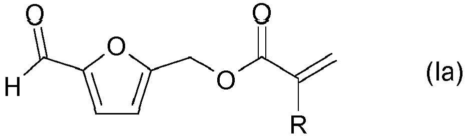 worin R n-Propyl, iso-Propyl, n-Butyl, iso-Butyl, sec-Butyl, tert-Butyl, n-Pentyl, isoPentyl, sec-Pentyl, tert-Pentyl, neo-Pentyl, Hexyl ist.

## Claims

1. A method for preparing a compound of the formula (I), 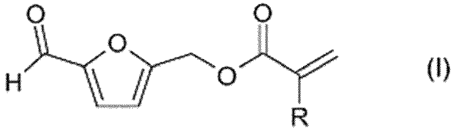 wherein R is H or C₁-C₆-alkyl,
by reacting at least one compound of the formula (II) 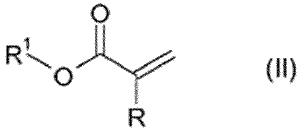
in which R has the same definition as in the formula (I) and
in which R¹ is H, C₁-C₁₂-alkyl or C₃-C₁₂-cycloalkyl,
with a compound of the formula (III) 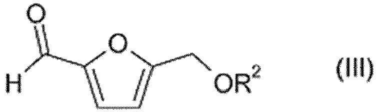
in which R² is H or C(O)R³,
wherein R³ is H or C₁-C₁₂-alkyl,
in the presence of at least one enzyme suitable for transesterification, wherein a lipase (EC 3.1.1.-) is used as enzyme.

2. The method according to claim 1, wherein R is H or CH₃.

3. The method according to claim 1 or 2, wherein R is H.

4. The method according to claim 1 or 2, wherein R is CH₃.

5. The method according to any of claims 1 to 4, wherein R¹ is CH₃ or CH₂CH₃.

6. The method according to any of claims 1 to 5, wherein R² is H.

7. The method according to any of claims 1 to 6, wherein the at least one compound of the formula (II) and the compound of the formula (III) are used in a molar ratio of from 5:1 to 15:1.

8. The method according to any of claims 1 to 7, wherein the method is carried out at temperatures in the range of 20 to 60°C.

9. A compound of the formula (Ia), 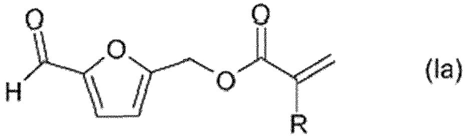 in which R is n-propyl, isopropyl, n-butyl, isobutyl, sec-butyl, tert-butyl, n-pentyl, isopentyl, sec-pentyl, tert-pentyl, neopentyl, hexyl.

## Revendications

1. Procédé de fabrication d'un composé de formule (I) 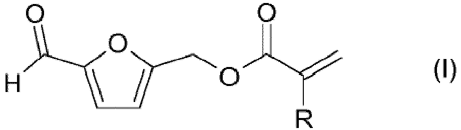 dans laquelle R représente H ou alkyle en C₁-C₆,
par mise en réaction d'au moins un composé de formule (II) 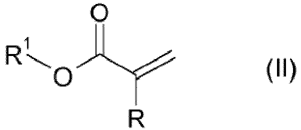
dans laquelle R a la même signification que dans la formule (I), et
dans laquelle R¹ représente H, alkyle en C₁-C₁₂ ou cycloalkyle en C₃-C₁₂,
avec un composé de formule (III) 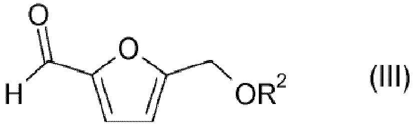
dans laquelle R² représente H ou C(O)R³,
dans laquelle R³ représente H ou alkyle en C₁-C₁₂,
en présence d'au moins une enzyme appropriée pour la transestérification, une lipase (EC 3.1.1.-) étant utilisée en tant qu'enzyme.

2. Procédé selon la revendication 1, dans lequel R représente H ou CH₃.

3. Procédé selon la revendication 1 ou 2, dans lequel R représente H.

4. Procédé selon la revendication 1 ou 2, dans lequel R représente CH₃.

5. Procédé selon l'une quelconque des revendications 1 à 4, dans lequel R¹ représente CH₃ ou CH₂CH₃.

6. Procédé selon l'une quelconque des revendications 1 à 5, dans lequel R² représente H.

7. Procédé selon l'une quelconque des revendications 1 à 6, dans lequel ledit au moins un composé de formule (II) et le composé de formule (III) sont utilisés en un rapport en moles de 5:1 à 15:1.

8. Procédé selon l'une quelconque des revendications 1 à 7, dans lequel le procédé est réalisé à des températures dans la plage allant de 20 à 60 °C.

9. Composé de formule (Ia) 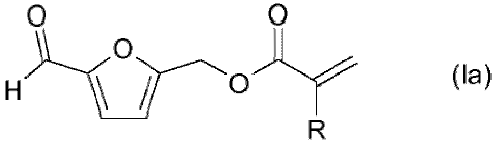 dans laquelle R représente n-propyle, iso-propyle, n-butyle, iso-butyle, sec-butyle, tert-butyle, n-pentyle, iso-pentyle, sec-pentyle, tert-pentyle, néopentyle, hexyle.
